(19) Europäisches Patentamt
European Patent Office
Office européen des brevets

(11) Publication number: **0 220 873 B1**

(12) **EUROPEAN PATENT SPECIFICATION**

(45) Date of publication of patent specification: **24.11.93**

(51) Int. Cl.[5]: **C07D 403/06**, C07D 403/14, C07D 491/18, A61K 31/415, A61K 31/495, A61K 31/50, A61K 31/505

(21) Application number: **86307953.9**

(22) Date of filing: **14.10.86**

The file contains technical information submitted after the application was filed and not included in this specification

(54) **Fused bicyclic imides with psychotropic activity.**

(30) Priority: **16.10.85 US 787887**
**30.07.86 US 892160**

(43) Date of publication of application:
**06.05.87 Bulletin 87/19**

(45) Publication of the grant of the patent:
**24.11.93 Bulletin 93/47**

(84) Designated Contracting States:
**AT BE CH DE ES FR GR IT LI LU NL SE**

(56) References cited:
**US-A- 4 543 355**
**US-A- 4 562 255**

**Chemical Abstracts, vol. 69, no.25, December 16, 1968, Colombus, Ohio, USA Gavrilov, L.D.; Vereschchagin, L.I.; Alesina, T.N. "Ouaternary ammonium salts of bis(3-azatricyclo-[5.3.0.1 1,5] undecane)" page 9978, column 2,abstract No 106 533r& Khim. Geterotsikl. Soedin. 1968, (3), 478-80**

(73) Proprietor: **AMERICAN HOME PRODUCTS CORPORATION**
**685, Third Avenue**
**New York, New York 10017(US)**

(72) Inventor: **Abou-Gharbia, Magid Abdel-Megid**
**2106 Weatherton Drive**
**Brandywood**
**Wilmington Delaware(US)**

(74) Representative: **Connelly, Michael John et al**
**John Wyeth & Brother Limited**
**Huntercombe Lane South**
**Taplow**
**Maidenhead Berkshire, SL6 0PH (GB)**

EP 0 220 873 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to new fused bicyclic imides that are useful as pharmaceuticals and in particular possess pyschotropic activity. The invention also provides pharmaceutical compositions containing the imides and a process for making the imides.

This invention provides novel compounds having antipsychotic activity and being characterised by the general formula

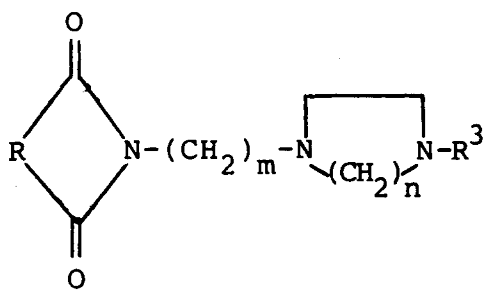

(I)

wherein

| | |
|---|---|
| R | represents formula III or $-CHR^1-CHR^2-$ in which |
| $R^1$ and $R^2$ | taken together represent formula II or IV |

(II) 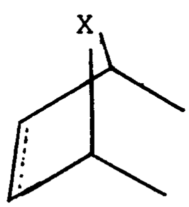 , (III) 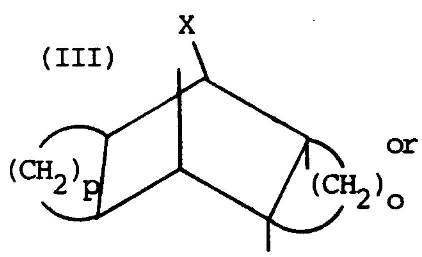 or (IV) 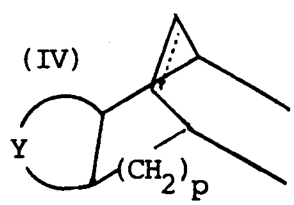

| | |
|---|---|
| $R^3$ | is unsubstituted or substituted phenyl, 2-pyridinyl, 2-pyrimidinyl, 2-pyrazinyl or 3-pyridazinyl, where the substituents are selected from the group lower alkyl, lower alkoxy, halo, cyano, nitro and trifluoromethyl, with the proviso that when $R^1$ and $R^2$ taken together represent |

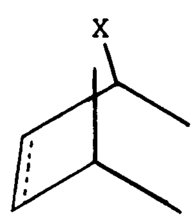

(II)

in which

| | |
|---|---|
| X | is O, methylene or dimethylene |
| $R^3$ | is other than unsubstituted or substituted phenyl, 2-pyridinyl or 2-pyrimidinyl; |
| Y | is $-(CH_2)_o-$ or vinylene; |
| X | is lower alkylene, vinylene or O; |
| m | is 2 - 4; |
| n | is 1 - 3; |
| o | is 0 - 3; |
| p | is 0 - 4; |
| ...... / ―― | represents a single or double bond; subject to the proviso that the sum of o and p in formula III and also in formula IV if Y is $-(CH_2)_o-$ is not zero; |

and the pharmaceutically acceptable salts thereof.

The term "lower alkyl" refers to moieties having 1-6 carbon atoms in the carbon chain. The term "lower alkylene" refers to moieties having 1-4 carbon atoms in the carbon chain. The term "alkoxy" refers to moieties having 1-6 carbon atoms. The term "halo" refers to fluoro, chloro and bromo.

The compounds of the invention can form pharmacologically acceptable salts from pharmacologically acceptable organic and inorganic acids such as hydrochloric; hydrobromic; sulphonic; sulphuric, phosphoric, nitric, maleic, fumaric, benzoic, ascorbic, pamoic, succinic, methanesulphonic, acetic, propionic, tartaric, citric, lactic, malic, mandelic, cinnamic, palmitic, itaconic and benzenesulphonic.

The compounds of the invention include those of formula I (in which $R^3$ is other than unsubstituted or substituted phenyl and R , m and n are as defined above save that p in formula III represents 1-4) and their pharmaceutically acceptable salts.

$R^3$ is preferably unsubstituted or substituted 2-pyrazinyl, 2-pyrimidinyl or 3-pyridazinyl advantageously unsubstituted or substituted 2-pyrazinyl or 3-pyridazinyl, where the substituents are as defined above.

In formulae herein a straight line interupted by $(CH_2)_p$ is equivalent to a direct bond if p is zero whereas a curved line interupted by $(CH_2)_o$ or $(CH_2)_p$ is equivalent to two hydrogen atoms if the number of methylene groups is zero.

The preparation of compounds having formula I will now be described. In this description the symbols $R,R^1$, $R^2$, $R^3$, X, Y, m, n , o, p and the dotted lines have the same meanings as given above unless otherwise stated. Moreover the symbol $A^1$ will be used to represent the group of general formula V and the symbol $A^2$ will be used to represent the group of general formula VI.

(V) 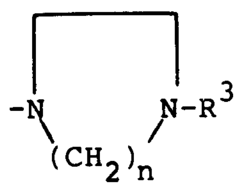

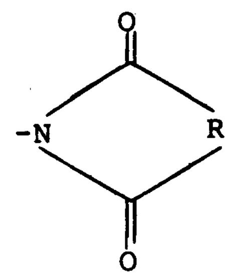 (VI)

The compounds having formula I and their salts may be prepared by a process in which

(a) a compound having the formula $A^1H$ or a salt thereof is N- alkylated to introduce the substituted alkyl group of formula $A^2-(CH_2)_m-$ by reaction with a compound having the formula $A^1-(CH_2)_m-Z^1$ (where $Z^1$ is a leaving group, for instance, a halogen atom such as chlorine or bromine or an organosulphonyloxy group, for example, methylsulphonyloxy or tosyloxy) as alkylating agent or by reductive alkylation with an aldehyde having the formula $A^2-(CH_2)_{m-1}-CHO$; or

(b) an imide having the formula $A^2H$ or a salt thereof is N- alkylated to introduce the substituted alkyl group of formula $A^1-(CH_2)_m-$ by reaction with a compound having the formula $A^1-(CH_2)_m-Z^2$ (where $Z^2$ is a leaving group, for example, a halogen atom such as chlorine or bromine) or a compound containing a cation having the formula VII

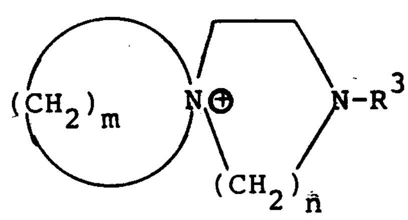 (VII)

as alkylating agent; or

(c) an amine having the formula $H_2N-(CH_2)_m-A^1$ or a salt or reactive derivative thereof is reacted with an acid having the formula $R(CO_2H)_2$ or a reactive derivative thereof to form an imide; or

(d) an amidic acid having the formula (VIII)

$CO_2H-R-CO-NH-(CH_2)_m-A^1$ (VII)

or a reactive derivative thereof is cyclised to form an imide; or

(e) an N-substituted maleimide having the formula IX

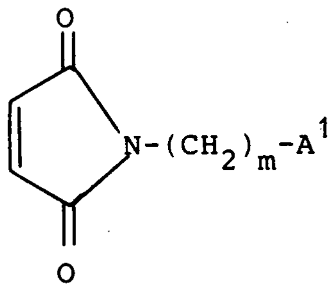

(IX)

is subjected to a Diels-Alder addition by reaction with a diene having the formula X, XI or XII

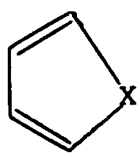

(X)

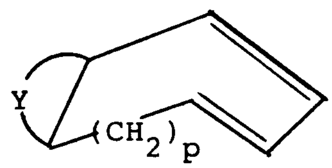

(XI)

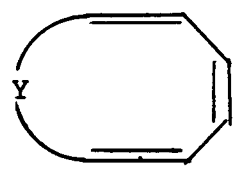

(XII)

(subject to the proviso that the diene is not benzene); or

(f) a compound having the formula XVI

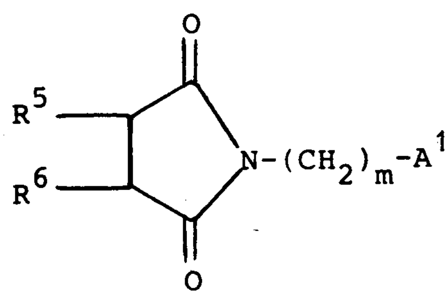

(XVI)

(wherein $R^5$ and $R^6$ together represent a group having the formula XVII, XVIII or XXI

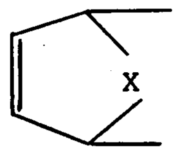

(XVII)

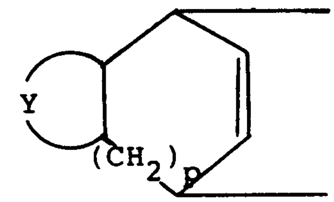

(XVIII)

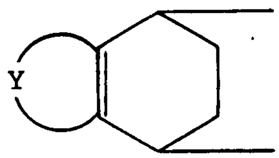

(XXI)

wherein p is not zero in formula XVIII if Y is $-(CH_2)_o$-where o is zero) or a salt thereof is reduced to convert an ethylenically unsaturated compound into a corresponding saturated compound; or

(g) a compound having formula I is converted into a salt thereof by addition of an acid or a salt of a compound having formula I is converted into a compound having formula I by subtraction of an acid.

Process step (a) can be carried out in known manner for converting secondary amines into tertiary amines. Preferably an imide having formula $A^2H$ is N-alkylated with a compound having the formula Hal-$(CH_2)_m$-Hal where each Hal is a halogen atom such as chlorine or bromine to form an N-substituted imide of the formula $A^2$-$(CH_2)_m$-Hal and this may then be used as alkylating agent for reaction with the compound having formula $A^1H$ to yield the final compound.

Process step (b) may be carried out in known manner for N-alkylation of imides. The alkylation is preferably carried out under basic conditions, for instance in the presence of sodium alkoxide, sodium hydride or sodamide so that the imide is present in salt form. The step (b) is preferably carried out by N-alkylation of the amine $A^1H$ with Hal-$(CH_2)_m$-Hal to yield Hal-$(CH_2)_m$-$A^1$ and use of this as alkylating agent for reaction with the imide having formula $A^2H$ which is preferably in salt form, to yield the final compound.

A number of procedures are available for preparing the final compounds by imide formation as in steps (c) and (d). The amine having the formula $H_2N$-$(CH_2)_m$-$A^1$ may be reacted with the dicarboxylic acid R-$(CO_2H)_2$ or a reaction derivative such as the anhydride or a diester. For instance one may carry out the synthesis:

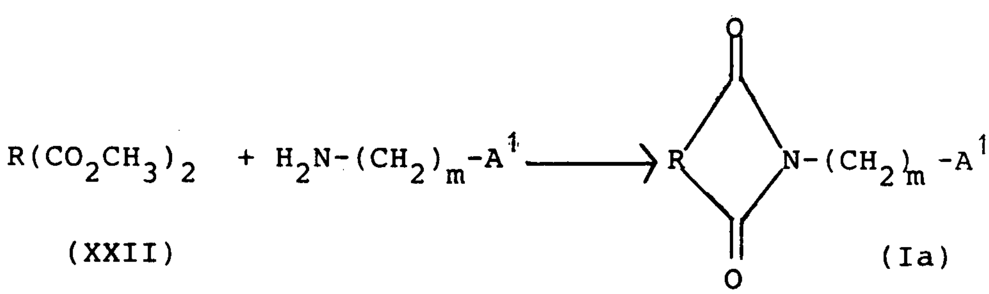

(XXII)

(Ia)

where $R^1$ and $R^2$ together represent a group of formula III. Instead of using the amine $H_2N$-$(CH_2)_m$-$A^1$ it may be possible to use a reactive derivative thereof. For instance the isocyanate (XXIV) may be reacted with the acid anhydride

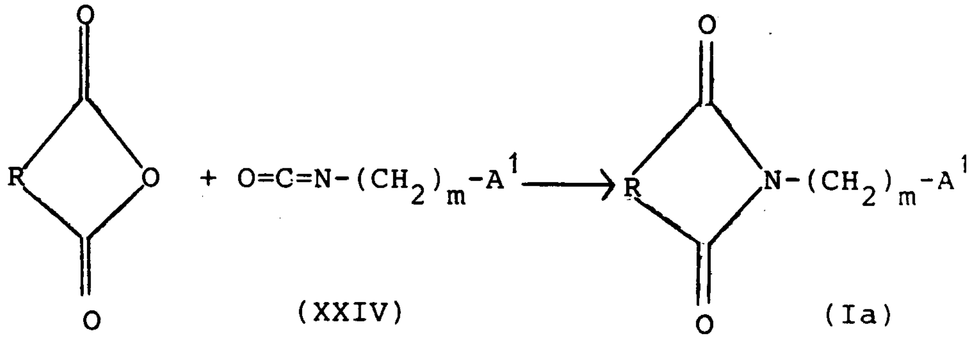

(XXIII) (XXIV) (Ia)

(XXIII) to yield the final compound (Ia).

Imide formation may also be carried out by cyclisation of the appropriate amidic acid (VIII) or a reactive derivative thereof. The amidic acid may be prepared where the reaction of the amine $H_2N\text{-}(CH_2)_m\text{-}A^1$ with the dicarboxylic acid $R(CO_2H)_2$ or reactive derivative thereof does not proceed fully to imide formation. Cyclisation of the amidic acid may be carried out by heating a mixture of the imidic acid, acetic anhydride and sodium acetate on a steam bath.

A number of starting materials for processes described above may be prepared from maleic acid or maleimide by means of a Diels-Alder addition. For instance reaction of maleimide (XXV) with a suitable diene, e.g. 1,3,5,7-cyclooctatetraene (XXVI)

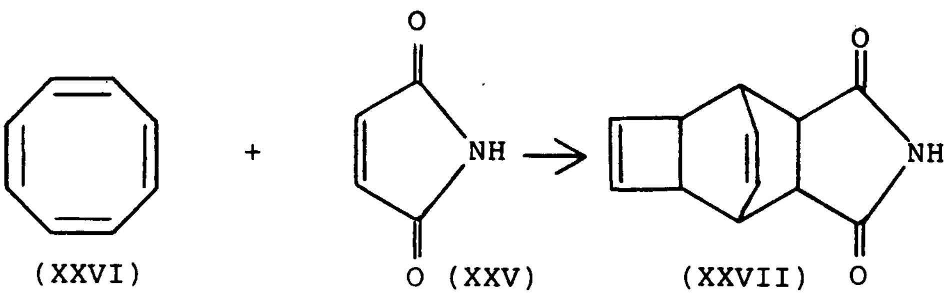

(XXVI) (XXV) (XXVII)

yields compounds $A^2H$, for instance, a compound (XXVII) which can be N-alkylated with a dihalo lower alkane to yield an alkylating agent (XXVIII) for reaction with the cyclic amine $A^1H$ to yield final compound (XXIX).

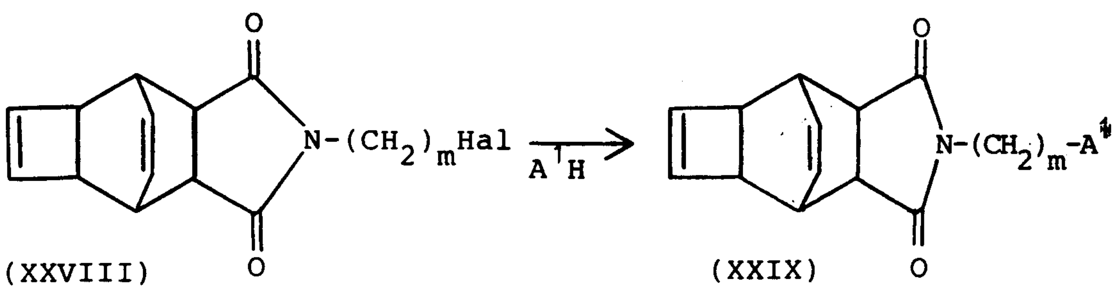

(XXVIII) (XXIX)

Compounds in which $R^1$ and $R^2$ taken together form a norbene ring can be prepared in a similar manner using norborn-5-ene-2,3-dicarboximide as the precursor which is reacted with the suitable dihalo lower alkane.

Compounds of the invention in which $R^1$ and $R^2$ taken together represent groups of formulae II and IV can be similarly prepared via the Diels-Alder addition outlined above, using a diene of appropriate ring size and degree of unsaturation such as 1,3,5-cycloheptatriene, 1,3-cyclooctadiene, 1,3,5-cycloocratriene and the like.

In an alternative preparative sequence, maleimide, can be first reacted with the dihalo lower alkane followed by reaction with the compound $A^1H$ to yield the intermediate N-substituted imide (IX). This can then be reacted with a suitable diene in accordance with the Diels-Alder addition [step (e)]. The reaction product of using a diene or formula X is a compound of formula I where $R^1$ and $R^2$ together represent a group of formula XVII. The reaction product of using a diene of formula XI is a compound where $R^1$ and $R^2$ is of formula XVIII. The reaction product of using a compound of formula XII is a compound in which $R^1$ and $R^2$ together form a group of formula XVIII in which p is zero.

In order to prepare final compounds where R is of formula III one preferably uses step (c), for example, using the dimethyl ester of formula $R(CO_2CH_3)_2$ .

The saturated analogues of ethylenically unsaturated compounds prepared in all the above reaction schemes may be prepared by reduction of the appropriate unsaturated intermediate or final product. In particular compound having formula XVI may be reduced [step (f)]. The reduction is preferably a hydrogenation method. The hydrogenation may be carried out using hydrogen and Pd/C as catalyst. The compounds having formula XVI may be prepared by a Diels-Alder addition using a suitable diene and a suitable dienophile as reactants. The compounds useful as reactants are as explained under step (e) or, in the case where R5 and R6 represent formula XXI, may be a compound of formula IX and a compound of formula XXXIV.

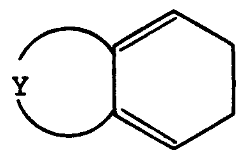

(XXXIV)

Where a compound having formula I has been prepared in free base form, a salt form may be prepared by addition of an acid. Where a compound having formula I has been prepared in the form of an acid addition salt, the free base form may be prepared by subtraction of the acid, for instance, by neutralisation with a base.

The starting materials used in the preparative routes described above are commercially available or can be made according to procedures taught in the chemical literature.

The invention also provides a pharmaceutical composition comprising a compound having formula I or a pharmaceutically acceptable salt therefrom in association or combination with a pharmaceutically acceptable carrier. The composition can be prepared by bringing the active compound into association or combination with the carrier.

The compounds of the invention display a pharmacological profile like that of the compound buspirone-(8-[4-[4-(2-pyrimidinyl)-1-piperazinyl]butyl]-8-azaspiro[4.5]decane-7,9-dione). The latter compound has demonstrated pre-clinical activity in antipsychotic paradigms and has also displayed a unique clinical anxioselective profile, whereby its efficacy in the treatment of anxiety neuroses is comparable to the benzodiazepine diazepam but without the benzodiazepine-related side effects. The clinically effective anxiolytic doses of the benzodiazepines produce such undesirable side effects as ataxia, muscle relaxation and sedation. Additionally. most chronically used antipsychotic drugs, cause extrapyramidal side effects, such as pseudoparkinsonism, tardive dyskinesia and the like. Ideally, treatment of psychoses and anxiety should be free of any undesirable side effects. The compounds of the invention, in a manner similar to buspirone, display preclinical antipsychotic activity without or with minimal side effects. Moreover, based on their buspirone-like profile, the compounds of the invention can be considered of clinical value in treating anxiety neuroses.

When employed as anxiolytics/antipsychotics, the effective dosage of the substances active for such treatment will vary according to the particular compound being employed, the severity and nature of condition being treated.
Therapy should be initiated at lower doses (in mg/kg/day), the dosage thereafter being increased, if necessary, to produce the desired effect. In general, the compounds of the invention are most desirably administered at a concentration that will generally afford effective results without causing any harmful or deleterious effects.

When the compounds of the invention are employed as anxiolytics/anti-psychotic agents, they can be formulated into oral dosage forms such as tablets, capsules and the like. The compounds can be administered alone or by combining them with conventional carriers, such as magnesium carbonate, magnesium stearate, talc, sugar, lactose, pectin, dextrin, starch, gelatin, tragacanth, methylcellulose, sodium

carboxymethylcellulose, low melting wax, cocoa butter and the like. Diluents, flavoring agents, solubilizers, lubricants, suspending agents, binders, tablet-disintegrating agents and the like may be employed. The compounds may be encapsulated with or without other carriers. In all cases, the proportion of active ingredients in said compositions both solid and liquid will be at least to impart the desired activity thereto on oral administration. The compounds may also be injected parenterally in which case they are used in the form of a sterile solution containing other solutes, for example, enough saline or glucose to make the solution isotonic.

The antipsychotic activity of the compounds of the invention and their substantial lack of extrapyramidal side effects may be demonstrated by standard pharmacological procedures, which are described more fully in the examples given hereafter.

The following examples show the preparation and pharmacological testing of compounds within the invention.

**Example 1**

**2-[4-[4-(6-Chloro-2-pyrazinyl)-1-piperazinyl]butyl]hexahydro-4,6-ethenocycloprop[f]isoindole-1,3-(2H,3aH)-dione, dihydrochloride, sesquihydrate**

To a stirred solution of 6.8 g (0.035 mol) of 1,3-dioxo-2H-4,6-etheno-1,3,3a,6a-tetrahydrocycloprop[f]-isoindole in 70 mL of dimethylformamide is added 0.9 g of sodium hydride. The suspension is stirred at 60 °C for 3 hrs and is poured into a stirred solution of 1,4-dibromobutane (9 g, 0.04 mol) in 50 mL of dimethylformamide.

The reaction mixture is stirred at room-temperature for 24 h, dimethylformamide is evaporated under reduced pressure and the residue is extracted with methylene chloride (3 x 200 mL). The methylene chloride extracts are collected, washed with water, dried over anhydrous $Na_2SO_4$ and evaporated under reduced pressure. The residue is solidified to a waxy like material affording 7.6 g (67% yield) of the corresponding 2-(4-bromobutyl)hexahydro-4,6-ethenocycloprop[f]isoindole-1,3(2H,3aH)-dione. The title compound is prepared by dissolving 2.5 g (0.007 mole) of 2-(4-bromobutylhexahydro-4,6-ethenocycloprop[f]-isoindole-1,3(2H,3aH)-dione in 50 mL of dimethylformamide, and to this solution is added 6 mL of triethylamine and 1.7 g (0.007 mL) of 1-(6-chloro-2-pyrazinyl)-piperazine hydrochloride. The reaction mixture is stirred at room temperature for 48 h. Dimethylformamide is removed under reduced pressure and the remaining solid is extracted with 2 x 100 mL of methylene chloride.

The methylene chloride extracts are dried over anhydrous $Na_2SO_4$,evaporated and the free base form of the title compound is separated by HPLC using 30% methanol-ethyl acetate as eluent. Evaporation of the solvent from the desired fractions($R_f$ 0.5) affords 0.95 g (31% yield) of the free base form of the title compound which is converted to the dihydrochloride salt by dissolving the free base in ethanol and adding ether saturated with hydrogen chloride; m.p. 258-261 °C.

| Analysis for: $C_{23}H_{28}ClN_5O_2$ • 2HCl • 1 1/2 $H_2O$ | | | |
|---|---|---|---|
| Calculated: | C, 50.96; | H, 6.09; | N, 12.92 |
| Found: | C, 50.90; | H, 6.09; | N, 13.09 |

**Example 2**

**4,4a,5,5a,6,6a-Hexahydro-2-[4-[4-(2-pyrimidinyl)-1-piperazinyl]butyl]-4,6-ethenocycloprop[f]isoindole-1,3-(2H,3aH)-dione,dihydrochloride, hydrate**

The free base form of the title compound is prepared following the procedure of Example 1 using 1-(2-pyrimidinyl)piperazine dihydrochloride instead of 1-(6-chloro-2-pyrazinyl)piperazine hydrochlorde and is converted to the dihydrochloride salt; m.p. 207-208 °C.

| Analysis for: $C_{23}H_{29}N_5O_2$ • 2HCl • $H_2O$ | | | | |
|---|---|---|---|---|
| Calculated: | C, 55.42; | H, 6.62; | N, 14.0; | C, 14.25 |
| Found: | C, 54.92; | H, 6.42; | N, 13.60; | Cl, 14.38 |

**Example 3**

**3a,4,4a,6a,7,7a-Hexahydro-2-[4-[4-(2-pyrimidinyl)-1-piperazinyl]butyl]-4,7-etheno-1H-cyclobut[f]-isoindole-1,3(2H)-dione, dihydrochloride, sesquihydrate**

The free base form of the title compound is prepared following the procedure of Example 1, using 1,3-dioxo-4,7-etheno- $\Delta^5$-1,3,3a,7a-tetrahydrocyclobut[f]isoindole instead of 1,3-dioxo-2H-4,6-etheno-1,3,3a,6a-tetrahydrocycloprop[f]isoindole, and 1-(2-pyrimidinyl)piperazine instead of 1-(6-chloro-2-pyrazinyl)piperazine. The compound is converted to the dihydrochloride salt; m.p. 252-254 °C.

| Analysis for: $C_{24}H_{29}N_5O_2$ • 2HCl • 1 1/2 $H_2O$ | | | |
|---|---|---|---|
| Calculated: | C, 55.44; | H, 6.54; | N, 13.48 |
| Found: | C,55.00; | H, 6.28; | N, 13.27 |

**Example 4**

**3a,4,4a,6a,7,7a-Hexahydro-2-[4-[4-(6-chloro-2-pyrazinyl)-1-piperazinyl]butyl]-4,7-etheno-1H-cyclobut-[f]isoindole-1,3(2H)-dione, dihydrochloride, sesquihydrate**

The free base form of the title compound is prepared following the procedure of Example 1 using 1-(6-chloro-2-pyrazinyl)piperazine hydrochloride instead of 1-(2-pyrimidyl)piperazine dihydrochloride and 1,3-dioxo-4,7-etheno-$^5$-1,3,3a,7a-tetrahydrocyclobut[f]isoindole instead of 1,3-dioxo-2H-4,6-etheno-1,3,3a,6a-tetrahydrocycloprop[f]isoindole. The compound is converted to the dihydrochloride salt; m.p. > 300 °C.

| Analysis for: $C_{24}H_{28}ClN_5O_2$ ● 2HCl ● 1 1/2 $H_2O$ | | | |
|---|---|---|---|
| Calculated: | C, 52.03; | H, 6.0; | N, 12.64 |
| Found: | C, 51.85; | H, 5.74; | N, 12.59 |

**Example 5**

**2-[4-[4-(6-Chloro-2-pyrazinyl-1-piperazinyl]butyl]octahydroo-4,7-ethano-1H-cyclobut[f]isoindole-1,3-(2H)-dione, hydrochloride, hemihydrate**

The free base form of the title compound is prepared following the procedure of Example 1 using 1,3-dioxo-4,7-ethano-1,3,3a,7a-tetrahydrocyclobut[f]isoindole instead of 1,3-dioxo-4,6-etheno-1,3,3a,6a-tetrahydrocycloprop[f]isoindole and is converted to the hydrochloride salt; m.p. 273-275 °C.

| Analysis for: $C_{24}H_{32}ClN_5O_2$ • HCl • 1/2 $H_2O$ | | | |
|---|---|---|---|
| Calculated: | C, 57.25; | H, 6.56; | N, 13.91 |
| Found: | C, 56.91; | H, 6.41; | N, 13.80 |

**Example 6**

**2-[4-[4-(2-Pyrimidinyl)-1-piperazinyl]butyl]octahydro-4,7ethano-1H-cyclobut[f]isoindole-1,3-(2H)-dione, dihydrochloride, sesquihydrate**

The free base form of the title compound is prepared following the procedure of Example 1 using 1-(2-pyrimidinyl)piperazine dihydrochloride instead of 1-(6-chloro-2-pyrazinyl)piperazine hydrochloride and 1,3-dioxo-4,7-etheno-1,3,3a,7a-tetrahydro-cyclobut[f]isoindole instead of 1,3-dioxo-2H-4,6-etheno-1,3,3a,6a-tetrahydro-cycloprop[f]isoindole. The compound is converted to the dihydrochloride salt; m.p. 237-240 °C.

| Analysis for: $C_{24}H_{35}N_5O_2$ • 2HCl • 1 1/2 $H_2O$ | | | |
|---|---|---|---|
| Calculated: | C, 55.06; | H, 7.26; | N, 13.38 |
| Found: | C, 55.15; | H, 6.87; | N, 13.34 |

**Example 7**

**2-[4-[4-(2-Pyrimidinyl)-1-piperazinyl]butyl]octahydro-4,6-ethanocycloprop[f]isoindole-1,3-(2H, 3aH)-dione, dihydrochloride, dihydrate**

The free base form of the title compound is prepared following the procedure of Example 1 using 1,3-dioxo-2H-4,6-ethano-1,3,3a,5a,6-tetrahydrocycloprop[f]isoindole instead of 1,3-dioxo-2H-4,6-etheno-1,3,3a,6a-tetrahydrocycloprop[f]isoindole and 1-(2-pyrimidinyl)piperazine dihydrochloride instead of 1-(6-chloro-2-pyrazinyl)piperazine hydrochloride. The compound is converted to the dihydrochloride salt; m.p. 190-192 °C.

| Analysis for: $C_{23}H_{31}N_5O_2$ • 2HCl • $2H_2O$ | | | |
|---|---|---|---|
| Calculated: | C, 53.24; | H, 7.13; | N, 13.50 |
| Found: | C, 53.41; | H, 6.70; | N, 13.43 |

**Example 8**

**2-[4-[4-(6-Chloro-2-pyrazinyl)-1-piperazinyl]butyl]octahydro-4,9-etheno-1H-cycloocta[c]pyrrole-1,3-(2H)-dione, dihydrochloride, sesquihydrate**

The free base form of the title compound is prepared following the procedure of Example 1 using octahydro-4,9-etheno-1H-cycloocta[c]pyrrole-1,3(2H)-dione instead of 1,3-dioxo-2H-4,6-etheno-1,3,3a,6a-tetrahydrocycloprop[f]isoindole and is converted to the hydrochloride salt; m.p. 248-250 °C.

| Analysis for: $C_{24}H_{32}ClN_5O_2$ • 2HCl • $1\frac{1}{2}H_2O$ | | | | |
|---|---|---|---|---|
| Calculated: | C, 51.65; | H, 6.80; | N, 12.55; | Cl, 19.41 |
| Found: | C, 51.13; | H, 5.70; | N, 13.16; | Cl, 20.63 |

**Example 9**

**2-[4-[4-(6-Chloro-3-pyridazinyl)-1-piperazinyl]butyl]hexahydro-4,6-ethenocycloprop[f]isoindole-1,3-(2H,3H)-dione, hydrochloride**

The free base form of the title compound is prepared following procedure of Example 1 using 1-(6-chloro-3-pyridazinyl)-piperazine instead of 1-(6-chloro-2-pyrazinyl)-piperazine and is converted to the hydrochloride salt; mp. 271-273 °C.

| Analysis for: $C_{23}H_{28}ClN_5O_2$ • HCl | | | |
|---|---|---|---|
| Calculated: | C, 57.74; | H, 6.06; | N, 14.64 |
| Found: | C, 57.61; | H, 6.01; | N, 14.30 |

**Example 10**

**2-[4-[4-(3-Chloro-2-pyrazinyl)-1-piperazinyl]butyl] octahydro-4,7-etheno-1H-cyclobut[f]isoindole-1,3-(2H)--dione, dihydrochloride, hydrate**

The title compound is prepared following the procedure of Example 1 using octahydro-4,7-etheno-1H--cyclobut[f]isoindole-1,3-(2H)-dione instead of hexahyro-4,6-ethenocycloprop[f]isoindole-1,3-(2H,3aH)--dione and 1-(3-chloro-2-pyrazinyl)piperazine instead of 1-(6-chloro-2-pyrazinyl)piperazine; and is converted to the hydrochloride salt; m.p. 208-210 ° C.

| Analysis for: $C_{24}H_{30}ClN_5O_2.2HCl.H_2O$ | | | |
|---|---|---|---|
| Calculated: | C,56.47; | H,6.47; | N,13.72 |
| Found: | C,55.94; | H,6.19; | N,13.43 |

**Example 11**

**3a,4,4a,6a,7,7a-Hexahydro-2-[4-[4-(6-chloro-3-pyridazinyl)-1-piperazinyl]butyl]4,7-etheno-1H--cyclobut[f]isoindole-1,3(2H)-dione, hydrochloride, hydrate**

The title compound is prepared following the procedure of Example 1 using 1,3-dioxo-4,7-etheno-Δ 5-1,3,3a,7a,-tetrahydrocyclobut[f]isoindole instead of hexahydro-4,6-ethenocycloprop[f]isoindole-1,3-(2H,3aH)--dione and 1-(6-chloro-3-pyridazinyl)-piperazine instead of 1-(6-chloro-2-pyrazinyl)-piperazine;and is converted to the hydrochloride salt; m.p.282-283 ° C.

| Analysis for: $C_{24}H_{28}ClN_5O_2$ . HCl . $H_2O$ | | | |
|---|---|---|---|
| Calculated: | C,56.72; | H,6.10; | N,13.78 |
| Found: | C,57.09; | H,5.90; | N,13.61 |

**Example 12**

**3a,4,4a,6a,7,7a-Hexahydro-2-[4-[4-(3-chloro-2-pyrazinyl-1-piperazinyl]butyl]4,7-etheno-1H-cyclobut[f]-isoindole-1,3(2H)-dione, hydrochloride, dihydrate**

The free base form of the title compound is prepared following the procedure of Example 1 using 1,3-dioxo-4,7-etheno- Δ 5-1,3,3a,7a,-tetrahydro-cyclobut[f]isoindole instead of hexahydro-4,6-ethenocycloprop[f]-isoindole-1,3-(2H,3aH)-dione and 1-(3-chloro-2-pyrazinyl)piperazine instead of 1-(6-chloro-2-pyrazinyl)-piperazine; and is converted to the hydrochloride salt; m.p. 150-152 ° C.

| Analysis for: $C_{24}H_{28}ClN_5O_2$ • $H_2O.2HCl$ | | | |
|---|---|---|---|
| Calculated: | C, 54.71; | H, 6.77; | N, 13.3 |
| Found: | C, 54.8; | H, 6.35; | N, 13.55 |

**Example 13**

**3a,4,7,7a,-Tetrahydro-2-[4-[4-(6-chloro-2-pyrazinyl)-1-piperazinyl] butyl]-4,7-methano-1H-isoindole-1,3(2H)-dione hydrochloride hydrate**

The free base form of the title compound is prepared using the procedure of Example 1 using norborn-5-ene-2,3-dicarboximide instead of 1,3-dioxo-2H-4,6-etheno-1,3,3a,6a-tetra-hydrocycloprop[f]isoindole and is converted to the hydrochloride salt; mp 235-237° C.

| Analysis for: $C_{21}H_{26}ClN_5O_2 \cdot HCl \cdot H_2O$ | | | |
|---|---|---|---|
| Calculated: | C, 53.61; | H, 6.17; | N, 14.89 |
| Found: | C, 53.04; | H, 6.07; | N, 15.47 |

**Example 14**

**2-[4-[4-(6-Chloro-2-pyrazinyl)-1-piperazinyl]butyl]octahydro-4,9-etheno-1H-cycloocta[c]pyrrole-1,3-dione, hydrochloride, hemihydrate**

The free base form of the title compound is prepared following the procedure of Example 1, using octahydro-4,9-ethano-1H-cycloocta[c]pyrrole-1,3(2H)-dione instead of hexahydro-4,6-ethenocycloprop[f]-isoindole-1,3(2H,3aH)-dione and is converted to the hydrochloride salt; ;m.p. 277-280° C.

| Analysis for: $C_{24}H_{34}ClN_5O_2 \cdot HCl \cdot 1/2\ H_2O$ | | | |
|---|---|---|---|
| Calculated: | C, 57.01; | H, 6.93; | N, 13.86 |
| Found: | C, 56.96; | H, 6.30; | N, 13.68 |

**Example 15**

**2-[4-[4-(6-Chloro-2-pyrazinyl)-1-piperazinyl]butyl]-octahydro-4,7-etheno-1H-cyclobut[f]isoindol-1,3-(2H)-dione, hydrochloride**

The free base form of the title compound is prepared following the procedure of Example 1, using octahydro-4,7-ethano-1H-cyclobut[f]isoindole-1,3(2H)-dione instead of hexahydro-4,6-ethenocycloprop[f]-isoindole-1,3(2H,3aH)-dione and is converted to the hydrochloride salt; m.p. 268-271° C.

| Analysis for: $C_{24}H_{30}ClN_5O_2 \cdot HCl$ | | | | |
|---|---|---|---|---|
| Calculated: | C, 58.53; | H, 6.30; | N, 14.22; | Cl, 14.43 |
| Found: | C, 59.03; | H, 6.36; | N, 14.12; | Cl, 13.86 |

**Example 16**

**2-[4-[4-(2-Pyrimidinyl)-1-piperazinyl]butyl]-octahydro-4,7-etheno-1H-cyclobut[f]isoindole-1,3-(2H)-dione, dihydrochloride, hydrate**

The free base form of the title compound is prepared following the procedure of Example 1, using 1-(2-pyrimidinyl)piperazine instead of 1-(6-chloro-2-pyrazinyl)piperazine hydrochloride and octahydro-4,7-etheno-1H-cyclobut[f]isoindole-1,3(2H)-dione instead of hexahydro-4,6-ethenocycloprop[f]isoindole-1,3(2H,3aH)-dione and is converted to the hydrochloride salt; m.p. 250-252° C.

| Analysis for: $C_{24}H_{31}N_5O_2 \cdot 2HCl \cdot H_2O$ | | | |
|---|---|---|---|
| Calculated: | C, 56.25; | H, 6.83; | N, 13.67 |
| Found: | C, 56.10; | H, 6.66; | N, 13.67 |

**Example 17**

**2-[4-[4-(6-Chloro-3-pyridazinyl-1-piperazinyl]butyl]-3a,4,7,7a-tetrahydro-4,7-methano-1H-isoindole-1,3-(2H)-dione, hydrochloride, dihydrate**

The free base form of the title compound is prepared following the procedure of Example 1, using 1-(6-chloro-3-pyridazinyl)piperazine hydrochloride instead of 1-(6-chloro-2-pyrazinyl)piperazine hydrochloride and 3a,4,7,7a-tetrahydro-4,7-methano-1H-isoindole-1,3(2H)-dione instead of hexahydro-4,6-ethenocycloprop[f]-isoindole-1,3(2H,3aH)-dione and is converted to the hydrochloride salt; m.p. 285°C.

| Analysis for: $C_{21}H_{26}N_5ClO_2 \cdot HCl \cdot 2\ H_2O$ | | | |
|---|---|---|---|
| Calculated: | C, 51.64; | H, 6.39; | N, 14.34 |
| Found: | C, 51.28; | H, 5.74; | N, 14.18 |

**Example 18**

**2-[4-[4-(3-Chloro-2-pyrazinyl)-1-piperazinyl]butyl]-3a,4,7,7a-tetrahydro-4,7-methano-1H-isoindole-1,3-(2H)-dione, hydrochloride, 2.5 hydrate**

The free base form of the title compound is prepared following the procedure of Example 1, using 1-(3-chloro-2-pyrazinyl)piperazine instead of 1-(6-chloro-2-pyrazinyl)piperazine hydrochloride and 3a,4,7,7a-tetrahydro-4,7-methano-1H-isoindole-1,3(2H)-dione instead of hexahydro-4,6-ethenocycloprop[f]isoindole-1,3-(2H,3aH)-dioneand is converted to the hydrochloride salt; m.p. 185-187°C.

| Analysis for: $C_{21}H_{26}N_5O_2Cl \cdot HCl \cdot 2.5\ H_2O$ | | | |
|---|---|---|---|
| Calculated: | C, 50.60; | H, 6.40; | N, 14.07 |
| Found: | C, 49.78; | H, 5.73; | N, 13.97 |

**Example 19**

**2-[4-[4-(3-Chloro-2-pyrazinyl)-1-piperazinyl]butyl]hexahydro-4,6-ethenocycloprop[f]isoindole-1,3-(2H,3aH)-dione, hydrochloride, hydrate**

The free base form of the title compound is prepared following the procedure of Example 1, using 1-(3-chloro-2-pyrazinyl)piperazine instead of 1-(6-chloro-2-pyrazinyl)piperazine hydrochloride and is converted to the hydrochloride salt; m.p. 110-112°C.

| Analysis for: $C_{23}H_{28}N_5O_2Cl \cdot HCl \cdot H_2O$ | | | |
|---|---|---|---|
| Calculated: | C, 55.64; | H, 6.29; | N, 14.11 |
| Found: | C, 55.56; | H, 6.21; | N, 14.16 |

**Example 20**

**2-[4-[4-(3-Chloro-2-pyrazinyl)-1-piperazinyl]butyl]octahydro-4,9-etheno-1H-cycloocta[c]pyrrole-1,3-(2H)-dione, hydrochloride, hydrate**

The free base form of the title compound is prepared following the procedure of Example 1, using octahydro-4,9-etheno-1H-cycloocta[c]pyrrole-1,3(2H)-dione instead of hexahydro-4,6-ethenocycloprop[f]-isoindole-1,3(2H,3aH)-dione and 1-(3-chloro-2-pyrazinyl)piperazine instead of 1-(6-chloro-2-pyrazinyl)-piperazine hydrochloride and is converted to the hydrochloride salt; m.p. 208-211 ° C.

| Analysis for: $C_{24}H_{32}ClN_5O_2$ • HCl • $H_2O$ | | | |
|---|---|---|---|
| Calculated: | C, 56.25; | H, 6.83; | N, 13.67 |
| Found: | C, 55.83; | H, 6.23; | N, 13.43 |

**Example 21**

**4,5,6,7,8,8a-Hexahydro-2-[4-[4-(2-pyrimidinyl)-1-piperazinyl]butyl]-4,8-ethenocyclohepta[c]pyrrole-1,3-(2H,3aH)-dione, dihydrochloride, hemihydrate**

The free base form of the title compound is prepared following the procedure of Example 1, using 4,5,6,7,8,8a-hexahydro-4,8-ethenocyclohepta[c]pyrrole-1,3(2H,3aH)-dione instead of hexahydro-4,7-ethenocycloprop[f]isoindole-1,3(2H,3aH)-dione and 1-(2-pyrimidinyl)piperazine instead of 1-(6-chloro-2-pyrazinyl)piperazine hydrochloride and is converted to the hydrochloride salt; m.p. 250-252 ° C.

| Analysis for: $C_{23}H_{31}N_5O_2$ • 2HCl • 1/2 $H_2O$ | | | | |
|---|---|---|---|---|
| Calculated: | C, 56.21; | H, 6.92; | N, 14.25; | Cl, 14.20 |
| Found: | C, 56.69; | H, 6.79; | N, 14.10; | Cl, 14.17 |

**Example 22**

**2-[4-[4-(6-Chloro-2-pyrazinyl)-1-piperazinyl]butyl]-4,5,6,7,8,8a-hexahydro-4,8-ethenocyclohepta[c]-pyrrole-1,3(2H,3aH)-dione, hydrochloride, hydrate**

The free base form of the title compound is prepared following the procedure of Example 1, using 4,5,6,7,8,8a-hexahydro-4,8-ethenocyclohepta[c]pyrrole-1,3(2H,3aH)-dione instead of hexahydro-4,7-ethenocycloprop[f]isoindole-1,3-(2H,3aH)-dioneand is converted to the hydrochloride salt; m.p. 254-256 ° C.

| Analysis for: $C_{23}H_{30}ClN_5O_2$ • HCl • $H_2O$ | | | |
|---|---|---|---|
| Calculated: | C, 55.53; | H, 6.63; | N, 14.08 |
| Found: | C, 55.69; | H, 6.77; | N, 14.88 |

**Example 23**

**3a,4,4a,6a,7,7a-Hexahydro-2-[4-[4-(2-pyrazinyl)-1-piperazinyl]butyl]-4,7-etheno-1H-cyclobut[f]-isoindole-1,3-(2H)-dione, fumarate(1:1)**

The free base form of the title compound is prepared following the procedure of Example 1, using 3a,4,4a,6a,7,7a-hexahydro-4,7-etheno-1H-cyclobut[f]isoindole-1,3(2H)-dione instead of hexahydro-4,7-ethenocycloprop[f]isoindole-1,3(2H,3aH)-dione and 1-(2-pyrazinyl)piperazine hydrochloride instead of 1-(6-chloro-2-pyrazinyl)piperazine hydrochloride and is converted to the fumarate salt; m.p. 169-170 ° C.

| Analysis for: $C_{24}H_{29}N_5O_2 \cdot C_4H_4O_4$ | | | |
|---|---|---|---|
| Calculated: | C, 62.80; | H, 6.16; | N, 13.08 |
| Found: | C, 63.00; | H, 6.35; | N, 13.00 |

**Example 24**

**4,4a,5,5a,6,6a-Hexahydro-2-[4-[4-(2-pyrazinyl)-1-piperazinyl]butyl]-4,6-ethenocycleprop[f]isoindole-1,3-(2H,3aH)-dione, dihydrochloride, hydrate**

The free base form of the title compound is prepared following the procedure of Example 1, using 1-(2-pyrazinyl)piperazine hydrochloride instead of 1-(6-chloro-2-pyrazinyl)piperazine hydrochloride and is converted to the hydrochloride salt; m.p. 200-202 °C.

| Analysis for: $C_{23}H_{29}N_5O_2 \cdot 2HCl \cdot H_2O$ | | | | |
|---|---|---|---|---|
| Calculated: | C, 55.42; | H, 6.67; | N, 14.05; | Cl, 14.23 |
| Found: | C, 55.38; | H, 6.67; | N, 13.86; | Cl, 14.30 |

**Example 25**

**2-[4-[4-(6-Chloro-2-pyrazinyl)-1-piperazinyl]butyl]hexahydro-4,6-ethanocycloprop[f]isoindole-1,3-(2H,3aH)-dione, hydrochloride**

The free base form of the title compound is prepared following the procedure of Example 1 using hexahydro-4,6-ethanocycloprop[f]isoindole-1,3-(2H,3aH)-dione instead of hexahydro-4,7-ethenocycloprop[f]-isoindole-1,3-(2H,3aH)-dioneand is converted to the hydrochloride salt; m.p. 266-268 °C.

| Analysis for: $C_{23}H_{30}ClN_5O_2 \cdot HCl$ | | | | |
|---|---|---|---|---|
| Calculated: | C, 57.50; | H, 6.45; | N, 14.58; | Cl, 14.79 |
| Found: | C, 57.33; | H, 6.75; | N, 14.08; | Cl, 14.40 |

**Example 26**

**2-[4-[4-(3-Chloro-2-pyrazinyl)-1-piperazinyl]butyl]hexahydro-4,6-ethanocycloprop[f]isoindole-1,3-(2H,3aH)-dione, hydrochloride**

The free base form of the title compound is prepared following the procedure of Example 1, using hexahydro-4,6-ethanocycloprop[f]isoindole-1,3-(2H,3aH)-dione instead of hexahydro-4,7-ethenocycloprop[f]-isoindole-1,3-(2H,3aH)-dioneand 1-(3-chloro-2-pyrazinyl)piperazine instead of 1-(6-chloro-2-pyrazinyl)-piperazine hydrochloride and is converted to the hydrochloride salt; m.p. 245-246 °C.

| Analysis for: $C_{23}H_{30}ClN_5O_2 \cdot HCl$ | | | | |
|---|---|---|---|---|
| Calculated: | C, 57.50; | H, 6.45; | N, 14.58; | Cl, 14.79 |
| Found: | C, 57.38; | H, 6.12; | N, 14.21; | Cl, 15.50 |

**Example 27**

**3a,4,4a,5,6,6a,7,7a-Octahydro-2-[4-[4-(2-pyrazinyl)-1-piperazinyl]butyl]-4,7-etheno-1H-cyclobut[f]-isoindole-1,3(2H)-dione, dihydrochloride, hemihydrate**

The free base form of the title compound is prepared following the procedure of Example 1, using 3a,4,4a,5,6,6a,7,7a-octahydro-4,7-etheno-1H-cyclobut[f]isoindole-1,3(2H)-dione instead of hexahydro-4,7-ethenocycloprop[f]isoindole-1,3(2H,3aH)-dione and 1-(2-pyrazinyl)piperazine hydrochloride instead of 1-(6-chloro-2-pyrazinyl)piperazine hydrochloride and is convrted to the hydrochloride salt; m.p. 208-210 °C.

| Analysis for: $C_{24}H_{31}N_5O_2$ • 2HCl • 1/2 $H_2O$ | | | |
|---|---|---|---|
| Calculated: | C, 57.25; | H, 6.75; | N, 13.91 |
| Found: | C, 57.12; | H, 6.88; | N, 13.86 |

**Example 28**

**3a,4,4a,6a,7,7a-Hexahydro-2-[4-[4-(5-trifluoromethyl-2-pyridinyl)-1-piperazinyl]-butyl]-4,7-etheno-1H-cyclobut[f]isoindole-1,3(2H)-dione, dihydrochloride, hydrate**

The free base form of the title compound is prepared following the procedure of Example 1, using 3a,4,4a,6a,7,7a-hexahydro-4,7-etheno-1H-cyclobut[f]isoindole-1,3(2H)-dione instead of hexahydro-4,7-ethenocycloprop[f]isoindole-1,3(2H,3aH)-dione and 1-(5-trifluoromethyl-2-pyridinyl)piperazine hydrochloride instead of 1-(6-chloro-2-pyrazinyl)piperazine hydrochloride and is converted to the hydrochloride salt; m.p. 233-235 °C.

| Analysis for: $C_{26}H_{29}F_3N_4O_2$ • 2HCl • $H_2O$ | | | |
|---|---|---|---|
| Calculated: | C, 54.03; | H, 5.71; | N, 9.69 |
| Found: | C, 54.17; | H, 5.58; | N, 9.86 |

**Example 29**

**4,5,6,7,8,8a-Hexahydro-2-[4-[4-(2-pyrimidinyl)-1-piperazinyl]butyl]-4,8-ethanocyclohepta[c]pyrrole-1,3-(2H,3aH)-dione, dihydrochloride**

The free base form of the title compound is prepared following the procedure of Example 1, using 4,5,6,7,8,8a-hexahydro-4,8-ethanocyclohepta[c]pyrrole-1,3(2H,3aH)-dione instead of hexahydro-4,7-ethenocycloprop[f]isoindole-1,3(2H,3aH)-dione and 1-(2-pyrimidinyl)piperazine instead of 1-(6-chloro-2-pyrazinyl)piperazine hydrochloride and is converted to the hydrochloride salt; m.p. 209-211 °C.

| Analysis for: $C_{23}H_{33}N_5O_2$ • 2HCl | | | |
|---|---|---|---|
| Calculated: | C, 57.02; | H, 7.23; | N, 14.46 |
| Found: | C, 57.74; | H, 7.23; | N, 14.50 |

**Example 30**

The compounds of the invention are tested in an assay to determine their ability to antagonise apomorphine-induced stereotyped behavior. The assay measures the in vivo dopamine reception blocking activity of the compounds and provides a means for gauging whether the compounds tested may potentially exhibit extrapyramidal side effects.

The assay is carried out as follows:

20-25 gm male CF-1 mice (Charles River) are used. The mice are tested one week before the

experiment for a positive stereotyped response to 10 mg/kg s.c. apomorphine. Test compounds, suspended or solubilized in 0.25% Tween 80® in water, are administered at several dose levels to male mice (6/dose level). A control group, run simultaneously with drug groups, receives equal volumes of solvent. Thirty minutes later (i.p. administration) or 60 minutes later (p.o. administration), drug-treated and control mice are challenged with 10 mg/kg apormorphine s.c.. Five minutes after the injection, the rearing-head-bobbing-licking syndrome induced by apomorphine is recorded as present or absent for each animal. Readings are repeated every 5 minutes during a 30 minute test session.

The number of positive or negative 5-minute intervals during which apomorphine-induced stereotyped behavior is present or absent is measured. $ED_{50}$ values (with 95% confidence intervals) are calculated for inhibition of apomorphine-induced stereotyped behavior, by a simple linear regression analysis with inverse prediction.

| Standard Compounds: | $ED_{50}$ and 95% confidence interval mg/kg intraperitoneal |
|---|---|
| Haloperidol | 1.37 ( 0.88- 2.34) |
| Chloropromazine | 8.48 ( 4.79-16.38) |
| Clozapine | 30.06 (19.42-48.21) |

The compounds of the invention, when tested in this assay are inactive, evidencing a low potential for exhibiting extrapyramidal side effects, such as pseudo parkinsonism, tardive dyskinesia and the like.

## Example 31

The compounds of the invention are further studied for their ability to inhibit limbic D-2 dopamine receptor binding. This in vitro assay measures the ability of the compounds tested to bind to the dopamine receptor sites. Those compounds which exhibit a weak binding effect have a low liability to display potential extrapyramidal side effects.

The test is carried out as follows:

Several rats are decapitated and the brains are rapidly removed. Limbic brain tissue (nucleus accumbens, septal area, olfactory tubercle) is dissected and homoganized on ice in 9 volumes of buffer (50 mM Tris-HCl, 120 mN NaCl, 5mM KCl, 1 mM $CaCl_2$, 1 mM $MgCl_2$, 0.1% L-ascorbic acid, 10 $\mu$M pargyline HCl, pH 7.1) using a Polytron homogenizer at setting 5 for three 15-sec bursts. The homogenate is then diluted 4-fold with buffer and centrifuged at 30,000 x g for 20 minutes, and the supernatant is discarded. The pellet is resuspended in the same volume of buffer and recentrifuged as before, again discarding the supernatant. This pellet is then resuspended in the same volume of buffer used in the homogenization, and the protein content of this preparation is assayed by the Lowry method. The homogenate is stored frozen at -70 °C until use.

Thirty $\mu$L of the homogenate (0.2-0.3 mg protein/sample) are incubated with 0.3 nM $^3$H-spiroperidol (New England Nuclear) and various concentrations of test drug in a final volume of 1 ml of the above buffer for 10 minutes in a 37 °C water bath. At the end of the incubation, 3 ml of cold 50 mM Tris-HC-l, pH 7.7, are added to each tube, and the contents are rapidly vacuum-filtered through Whatman GF/B glass-fiber filters. The filters are then rapidly washed 3 times with 3 ml of the same buffer, placed in scintillation vials, and shaken for 15 minutes with 10 ml of Hydrofluor (National Diagnostics) scintillation cocktail. The vials are then counted in a Packard 460CD scintillation counter.

Specific binding is defined as total binding less binding in the presence of 1$\mu$M (+)butaclamol. Binding in the presence of various concentrations of test drug is expressed as a per cent of specific binding when no drug is present. These results are then plotted as logit % binding vs. log concentration of test drug. Linear regression analysis then yields a straight line with 95% confidence limits from which an $IC_{50}$ can be inversely predicted. $K_i$ (inhibition constant) for the test drug is then calculated by the formula:

$$K_i = \frac{IC_{50}}{1 + \frac{[^3H\text{-Spiroperidol}]}{K_D}}$$

where $K_D$ = 0.3 nM for spiroperidol binding

| Standard Compounds: | $K_i$ and 95% confidence interval |
|---|---|
| Haloperidol | 4.0 (3.0 - 5.6) nM |
| Clozapine | 34 (23 - 54) nM |
| Fluphenazine | 4.5 (3.6 - 5.6) nM |
| Sulpiride | 376 (174 - 5000) nM |

The results of testing of some of the compounds of the invention, and the prior art compound buspirone (8-[4-[4-(2-pyrimidinyl)-1-piperazinyl]butyl]-8-azapiro[4.5]-decane-7,9-dione) in this assay are presented in Table 1.

**Table 1**

| Compound of Example No. | Limbic D-2 Binding ($K_i$nM) |
|---|---|
| Buspirone | 119 |
| 1 | 690 |
| 3 | 345 |
| 7 | 140 |
| 8 | 345 |
| 13 | 1% inhibition at 1 μM |
| 14 | 440 |
| 15 | 413 |
| 16 | 85 |
| 17 | 19% inhibition at 1 μM |
| 18 | 48% inhibition at 1 μM |
| 19 | 54 |
| 20 | 78% inhibition at 1 μM |
| 21 | 53% inhibition at 1 μM |
| 22 | 43% inhibition at 1 μM |
| 23 | 13% inhibition at 1 μM |
| 24 | 38% inhibition at 1 μM |
| 25 | 49% inhibition at 1 μM |
| 26 | 75% inhibition at 1 μM |
| 27 | 41% inhibition at 1 μM |

The results show that compounds of the invention display a very weak effect, evidencing a low potential for extrapyramidal side effects.

## Example 32

The antipsychotic activity of the compounds of the invention is assessed via the conditioned avoidance (discrete trial) test. This test has excellent clinical correlation for antipsychotic activity.

The test is carried out as follows:

Male CD rats (Charles River) maintained at approximately 400-450 gm body weight are used. Rats trained previously are placed in plexiglass experimental chambers equipped with a response lever, house light, and sonalert. A steel grid floor is wired for presentation of electric shock. Each trial consists of a fifteen-second warning tone (conditioned stimulus), continuing for an additional fifteen seconds accompanied by electric shock, (unconditioned stimulus). The rat can terminate a trial at any point by depression of the response lever. As response during the initial fifteen-second warning tone ends the trial before shock delivery and is considered avoidance response, while a response occurring during shock delivery is an escape response. Trials are presented on a variable interval schedule of two minutes. The session consists of sixty trials. Animals are run two to three times weekly with control sessions always preceding a drug run,

and with at least one day intervening, compounds are administered i.p. or p.o. at appropriate pre-treatment times to a minimum of five to six rats at each dose level over a range of doses.

The following experimental parameters are recorded by computer: (1) the number of interval responses, (2) the number of avoidance responses, (3) the number of escape responses, and (4) the number of trials in which no response occurred. These data are used to calculate the percent difference from control values previously determined and are presented for visual comparison via a line graph.

Response counts are summed over all subjects at a given dose. The number of trials in which rats fail to exhibit an avoidance response (Avoidance Block, AB) is determined at each dose. This number is expressed as a percentage of the total trials. Control performance is arbitrarily assigned a value of 100% for avoidance and the dose calculated to produce a 50% block in avoidance responding ($AB_{50}$) is obtained from a dose-effect regression line fitted by the method of least squares. Potential antipsychotic compounds suppress avoidance responding and increase escape responding.

| STANDARD COMPOUNDS | $AB_{50}$ (mg/kg i.p.) |
|---|---|
| Spiperone | 0.13 |
| Haloperidol | 0.18 |
| Chlorpromazine | 2.50 |
| Thioridazine | 8.61 |
| Clozapine | 10.82 |

The results for compounds of this invention in this test are presented in Table 2.

**Table 2**

| Compound of Example No. | Active at mg/kg |
|---|---|
| 1 | 40 (p.o.)*[1] |
| 2 | 40 (intraperitoneally administered) |
| 3 | 13.4 (p.o.)[1] |
| 6 | 40 (p.o.) |
| 28 | 40 (p.o.) |
| 13 | 39.7 (p.o.)[1] |
| 15 | 40 (p.o.) |
| 16 | 47.4 (p.o.)[1] |
| 17 | 40 (p.o.) |
| 18 | 40 (p.o.)[1] |
| 20 | 40 (p.o.) |
| 22 | 68.4 (p.o.)[1] |
| 23 | 40 (p.o.) |
| 24 | 49.58 (p.o.)[1] |
| 25 | 40 (p.o.) |
| 26 | 40 (p.o.) |
| 27 | 40 (p.o.) |

* (p.o.) = perorally administered drug.
[1] = $AB_{50}$ value

The results show that compounds of the invention have significant oral activity in the test procedure employed.

## Example 33

Another test designed to determine the potential antipsychotic activity of the compounds of the invention is the conditioned avoidance (shelf-jump response) test.

This test is carried out as follows:

Male CD rats (Charles River) maintained at approximately 400-450 gm body weight are used. Previously trained rats are placed in plexiglass experimental chambers divided into two sections; a main chamber (10 1/2" x 6 3/4" x 11 7/8" high) and an elevated chamber or shelf (5 7/8" x 6 7/8" x 5 3/4"). A moveable wall, controlled by a motor, determines whether the rat has access to the shelf at any time during the experiment. The experimental chamber also contains a house light and sonalert. A steel grid floor in the main chamber is wired for presentation of electric shock. Each trial consists of a fifteen-second warning tone (conditioned stimulus), continuing for an additional fifteen seconds accompanied by electric shock, (unconditioned stimulus). A response (jumping onto the exposed shelf of the upper chamber) occurring during the initial fifteen-second warning tone is considered an avoidance response, while a response occurring during shock delivery is considered an escape response. Trials are presented on a fixed interval schedule of one minute. The session consists of thirty-six trials. Animals are run twice weekly with control sessions always preceding a drug run, and with at least one day intervening. Compounds are administered i.p. or p.o. at appropriate pre-treatment times to a minimum of five rats at each dose level over a range of doses.

The following experimental parameters are recorded by computer: (1) the number of avoidance responses, (2) the number of escape responses, and (3) the number of trials in which no response occurred. These data are used to calculate the percent difference from control values previously determined and are presented for visual comparison via a line graph.

Response counts are summed over all subjects at a given dose. The number of trials in which rats fail to exhibit an avoidance response (Avoidance Block, AB) is determined at each dose. This number is expressed as a percentage of the total trials. Control performance is arbitrarily assigned a value of 100% for avoidance and the dose calculated to produce a 50% block in avoidance responding ($AB_{50}$) is obtained from a dose-effect regression line fitted by the method of least squares. Potential antipsychotic compounds suppress avoidance responding and increase escape responding.

| Standard Compounds: | $AB_{50}$ (mg/kg i.p.) |
|---|---|
| Haloperidol | 0.19 |
| Chlorpromazine | 3.69 |
| Clozapine | 6.94 |
| Buspirone | 9.94 |

The results for compounds of this invention in this test are presented in Table 3.

**Table 3**

| Compound of Example No. | Active at mg/kg |
|---|---|
| 1 | 40 (i.p.)* |
| 2 | 40 (i.p.) |
| 3 | 40 (i.p.) |
| 4 | 40 (i.p.) |
| 5 | 40 (i.p.) |
| 6 | 40 (i.p.) |
| 7 | 40 (i.p.) |
| 8 | 40 (i.p.) |
| 9 | 40 (i.p.) |

**Table 3 (continued)**

| Compound of Example No. | Active at mg/kg |
|---|---|
| 10 | 40 (i.p.) |
| 11 | 40 (i.p.) |
| 12 | 40 (i.p.) |
| 13 | 40 (i.p.) |
| 14 | 40 (i.p.) |
| 15 | 40 (i.p.) |
| 16 | 40 (i.p.) |
| 17 | 40 (i.p.) |
| 18 | 40 (i.p.) |
| 19 | 40 (i.p.) |
| 20 | 40 (i.p.) |
| 21 | 40 (i.p.) |
| 22 | 40 (i.p.) |
| 23 | 40 (i.p.) |
| 24 | 40 (i.p.) |
| 25 | 40 (i.p.) |
| 26 | 40 (i.p.) |
| 27 | 40 (i.p.) |
| 28 | 40 (i.p.) |
| 29 | 40 (i.p.) |

*** (i.p.) = intraperitoneally administered drug.**

The results show that compounds of the invention are active intraperitoneally in this test.

**Example 34**

The ex vivo inhibition of 5-HT-1A serotonin receptor binding assay is used to determine whether the test compounds can cross the blood-brain barrier and affect the receptor in question and to give an indication of buspirone-like anxiolytic activity.

The assay is carried out as follows:

Several groups of rats (4 - 6 rats/group) are injected with test compound or the appropriate vehicle. Thirty minutes later, unless otherwise noted, rats are decapitated and their brains removed. Various brain regions are dissected and rapidly frozen and maintained at -70 °C until used.

Hippocampal tissue is dissected and homogenized on ice in 40 vols of buffer (50 mM Tris HCl, pH = 7.7) using a Polytron homogenizer at setting 5 for 3 x 15 sec bursts. The homogenate is then centrifuged at 20,000 rpm (RC5-B; 50,000 g) and the supernatant discarded. The pellet is resuspended in 40 vols of the same buffer and incubated at 37 °C for 10 minutes to aid in the removal of endogenous serotonin. The homogenate is then centrifuged (as above) and the supernatant discarded. The pellet is then resuspended in 100 vols of buffer B (50 mM Tris HCl, pH = 7.7 containing 0.1% ascorbate, 10 µM pargyline and 4 mM $CaCl_2$) and sonicated. An aliquot is taken for protein determination by the Lowry method and the remainder stored frozen at -70 °C until used.

The homogenate (50 µl; 0.4-0.6 mg protein/sample) is incubated with 100 µl (1.5-1.8 nM) $^3$H-8-hydroxy-2-(di-n-propylamino)tetraline in a final volume of 2 ml of buffer for 10 minutes at 37 °C. At the end of the incubation, 3 ml of cold buffer A are added to each tube, and the contents rapidly filtered through Whatman GF/B glass filters. The filters are then rapidly washed 2 times with 3 ml of the same buffer, placed in scintillation vials, and shaken for 15 minutes with 10 ml of Hydrofluor (National Diagnostics)scintillation cocktail. The vials are then counted in a Packard 460 CD scintillation counter.

Specific binding is calculated for each of the treatment protocols and is defined as total binding less binding in the presence of excess unlabeled serotonin (1 µM). Specific binding obtained in vehicle-treated

rats is compared to that obtained in animals receiving a single or various doses of test compound and expressed as percent of control. These results are then plotted as logit % binding vs. log concentration of test drug. Linear regression analysis then yields a straight line with 95% confidence limits from which an $IC_{50}$ can be inversely predicted. Ki (inhibition constant) for the test drug is then calculated by the formula:

$$K_i = \frac{IC_{50}}{1 + \frac{[^3H\text{-}8\text{-}OH\ DPAT]}{K_D}}$$

where $K_D$ = 1.8 nm for 8-OH DPAT binding in hippocampus

The use of several doses of test compound also permits the calculation of an $ID_{50}$ value, i.e. an inhibitory dose that displaces 50% of the specific binding ex vivo.

Under these conditions, buspirone (30 mg/kg) displaced 46% of specific $^3$H-8-OH-DPAT binding from hippocampal membranes.

When tested in this assay, the compounds of the invention gave the following results.

Table 4

| Compound of Example No. | Inhibition Constant ($K_i$ nm) |
|---|---|
| 1 | 162 |
| 3 | 17 |
| 7 | 13.9 |
| 8 | 122 |
| 13 | 74% inhibition at 1 $\mu$m |
| 16 | 1.1 |
| 21 | 12 |
| 22 | 107 |
| 23 | 46% inhibition at 1 $\mu$m |
| 24 | 68% inhibition at 1 $\mu$m |
| 27 | 41 |

The results show that compounds of the invention have a moderate to extremely strong affinity for binding to the 5-HT-1A receptor site, evidencing a high potential for anxiolytic activity.

US 4,543, 355 discloses succinimide derivatives useful as antianxious substances. The compounds include those of formula I where $R^3$ is phenyl or substituted phenyl, 2-pyridyl or 2-pyrimidinyl, n is 2, m is 3 or 4 and R is -$CHR^1$-$CHR^2$- where $R^1$ and $R^2$ represent formula II in which X is O, methylene or ethylene.

**Claims**

**Claims for the following Contracting States : BE, CH, DE, FR, IT, LI, LU, NL, SE**

**1.** A compound having the formula

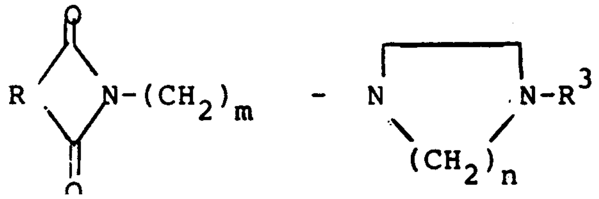

wherein R represents formula III or represents -$CHR^1$-$CHR^2$- where $R^1$ and $R^2$ taken together represent formula II or IV

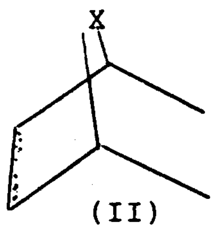
(II)

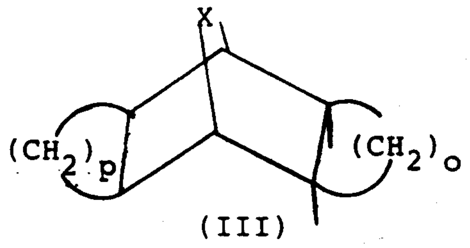
(III)

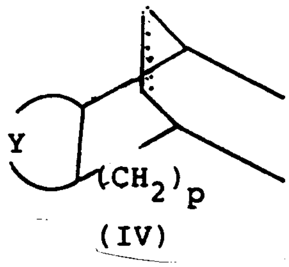
(IV)

$R^3$ is unsubstituted or substituted phenyl, 2-pyridinyl, 2-pyrimidinyl, 2-pyrazinyl, or 3-pyridazinyl; where the substituents are selected from the group $C_1$-$C_6$ alkyl, $C_1$-$C_6$ alkoxy, halo, cyano, nitro and trifluoromethyl with the proviso that when $R^1$ and $R^2$ taken together represent formula II in which X is O, methylene or dimethylene, $R^3$ is other than unsubstituted or substituted phenyl, 2-pyridinyl or 2-pyrimidinyl;

Y is $-(CH_2)_o-$ or vinylene;
X is $C_1$-$C_4$ alkylene, vinylene or O;
m is 2 - 4;
n is 1 - 3;
o is 0 - 3;
p is 0 - 4;
..... represents a single or double bond; subject to the proviso that the sum of o and p in formula III and in formula IV where Y is $-(CH_2)_o-$ is not zero; or a pharmaceutically acceptable salt thereof.

**2.** A compound as claimed in Claim 1, wherein $R^3$ is unsubstituted or substituted 2-pyrazinyl or 3-pyridazinyl, the substituents being as defined in Claim 1.

**3.** A compound as claimed in Claim 1, wherein R represents $-CHR^1$-$CHR^2$- where $R^1$ and $R^2$ together represent formula IV as defined and illustrated in Claim 1 and o is other than zero.

**4.** 2-[4-[4-(6-Chloro-2-pyrazinyl)-1-piperazinyl]butyl] hexahydro-4,6-ethenocycloprop[f]isoindole-1,3-(2H,3aH)-dione or a pharmaceutically acceptable salt thereof.

**5.** 4,4a,5,5a,6,6a-Hexahydro-2-[4-[4-(2-pyrimidinyl)-1-piperazinyl]butyl]-4,6-ethenocycloprop[f]isoindole-1,3-(2H,3aH)-dione or a pharmaceutically acceptable salt thereof.

**6.** 3a,4,4a,6a,7,7a-Hexahydro-2-[4-[4-(2-pyrimidinyl)-1-piperazinyl]butyl]-4,7-etheno-1H-cyclobut[f]-isoindole-1,3-(2H)-dione or a pharmaceutically acceptable salt thereof.

**7.** A compound selected from 3a,4,4a,6a,7a-hexahydro-2-[4-[4-(6-chloro-2-pyrazinyl)-1-piperazinyl]butyl]-4,7-etheno-1H-cyclobut[f]isoindole-1,3(2H)-dione, 2-[4-[4-(6-chloro-2-pyrazinyl)-1-piperazinyl]butyl]-octahydro-4,7-ethano-(1H-cyclobut[f]isoindole-1,3-(2H)-dione,2-[4-[4-(2-pyrimidinyl)-1-piperazinyl]butyl]-octahydro-4,7-ethano-1H-cyclobut[f]isoindole-1,3-(2H)-dione, 2-[4-[4-(2-pyrimidinyl) -1-piperazinyl]butyl]-octahydro-4,6-ethanocycloprop[f]-isoindole-1,3-(2H, 3aH)-dione, 2-[4-[4-(6-chloro-2-pyrazinyl)-1-piperazinyl]butyl]octahydro-4,9-etheno-1H-cycloocta[c] pyrrole-1,3-(2H)-dione, 2-[4-[4-(6-chloro-3-pyridazinyl)-1-piperazinyl]butyl]hexahydro-4,6-ethenocycloprop[f] isoindole-1,3-(2H,3H)-dione, 2-[4-[4-(3-chloro-2-pyrazinyl)-1-piperazinyl]butyl]octahydro-4,7-etheno-1H-cyclobut[f] isoindole-1,3-(2H)-dione, 3a,4,4a,6a,7,7a-hexahydro-2-[4-[4-(6-chloro-3-pyridazinyl)-1-piperazinyl]butyl]-4,7-etheno-1H-cyclobut[f]-isoindole-1,3-(2H)-dione, 3a,4,4a,6a,7,7a-hexahydro-2-[4-[4-(3-chloro-2-pyrazinyl)-1-piperazinyl] butyl]-4,7-etheno-1H-cyclobut[f]isoindole-1,3-(2H)-dione, 3a,4,7,7a-tetrahydro-2-[4-[4-(6-chloro-2-pyrazinyl)-1-piperazinyl]butyl]-4,7-methano-1H-isoindole,2-[4-[4-(6-chloro-2-pyrazinyl)-1-piperazinyl]butyl]octahydro-4,9-ethano-1H-cycloocta[c]pyrrole-1,3-dione, 2-[4-[4-(6-chloro-2-pyrazinyl)-1-piperazinyl]butyl]-octahydro-4,7-etheno-1H-cyclobut[f]isoindol-1,3-(2H)-dione, 2-[4-[4-(2-pyrimidinyl)-1-piperazinyl]butyl]-octahydro-4,7-etheno-1H-cyclobut[f]isoindole-1,3-(2H)-dione,2-[4-[4-(6-chloro-3-pyridazinyl)-1-piperazinyl]butyl]-3a,4,7,7a-tetrahydro-4,7-methano-1H-isoindole-1,3-(2H)-dione, 2-[4-[4-(3-chloro-2-pyrazinyl)-1-piperazinyl]butyl]-3a,4,7,7a-tetrahydro-4,7-methano-1H-isoindole-1,3-(2H)-dione, 2-[4-[4-(3-chloro-2-pyrazinyl)-1-piperazinyl]butyl]hexahydro-4,6-ethenocycloprop[f]isoindole-1,3-(2H,3aH)-dione, 2-[4-[4-(3-chloro-2-pyrazinyl)-1-piperazinyl]butyl]octahydro-4,9-etheno-1H-cycloocta[c]pyrrole-1,3(2H)-dione, 4,5,6,7,8,8a-hexahydro-2-[4-[4-(2-pyrimidinyl)-1-piperazinyl]butyl]-4,8-ethenocyclohepta[c]pyrrole-1,3-(2H,3aH)-dione, 2-[4-[4-(6-chloro-2-pyrazinyl)-1-piperazinyl]butyl]-4,5,6,7,8,8a-hexahydro-4,8-ethenocyclohepta[c]-pyrrole-1,3(2H,3aH)-dione, 3a,4,4a, 6a,7,7a-hexahydro-[2-[4-[4-(2-pyrazinyl)-1-piperazinyl ]butyl]-4,7-etheno-1H-cyclobut[f]isoindole-1,3-(2H)-dione, 4,4a,5,5a,6,6a-hexahydro-2,[4-[4-(2-pyrazinyl)-1-piperazinyl]butyl]-4,6-ethenocycloprop[f]isoindole-1,3-(2H,3aH)-dione,2-[4-[4-(6-chloro-2-pyrazinyl)-1-piperazinyl] butyl]hexahydro-4,6-ethanocycloprop[f]isoindole-1,3-(2H,3aH)-dione, 2-[4-[4-(3-chloro-2-pyrazinyl)-1-piperazinyl]butyl]hexahydro-4,6-ethanocycloprop[f]isoindole-1,3-(2H,3aH)-dione,3a,4,4a,5,6,6a,7,7a-octahydro-2-[4-[4-(2-pyrazinyl)-piperazinyl]butyl]-4,7-etheno-1H-cyclobut[f]-isoindole-1,3(2H)dione, 3a,4,4a,6a,7,7a-hexahydro-2-[4-[4-(5-trifluoromethyl-2-pyridinyl)-1-piperazinyl]-butyl]4,7-etheno-1H-cyclobut[f]isoindole-1,3(2H)-dione,4,5,6,7,8,8a-hexahydro-2-[4-[4-(2-pyrimidinyl)-1-piperazinyl]butyl]-4,8-ethanocyclohepta[c]pyrrole-1,3-(2H,3aH)-dione and their pharmaceutically acceptable salts.

**8.** A pharmaceutical composition comprising a compound as claimed in any one of Claims 1 to 7 in association or combination with a pharmaceutically acceptable carrier.

**9.** A pharmaceutical composition as claimed in Claim 8 in unit dosage form as an antipsychotic agent.

**10.** A process for the preparation of a compound having the formula $A^1$-$(CH_2)_m$-$A^2$ wherein $A^1$ represents a group having the formula V

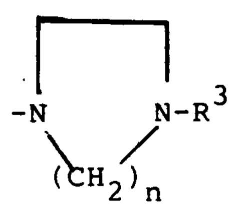

(V)

where $R^3$ and n are as defined in Claim 1, m is as defined in Claim 1 and $A^2$ represents formula VI

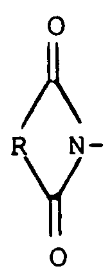

(VI)

where R is as defined in Claim 1 or a salt thereof, wherein

(a) a compound having the formula $A^1$-H (where $A^1$ is as defined above) or a salt thereof is N-alkylated to introduce a substituted alkyl group by reaction with a compound having the formula $A^2$-$(CH_2)_m$-$Z^1$ as alkylating agent (where $A^2$ and m are as defined above and $Z^1$ is a leaving group) or by reductive alkylation with an aldehyde having the formula $A^2$-$(CH_2)_{m-1}$-CHO (where $A^2$ and m are as defined above); or

(b) an imide having the formula $A^2$H (where $A^2$ is as defined above) or a salt thereof is N-alkylated to introduce a substituted alkyl group by reaction with a compound having the formula $A^1$-$(CH_2)_m$-$Z^2$ where $A^1$ and m are as defined above and $Z^2$ is a leaving group or with a compound containing the cation having the formula VII

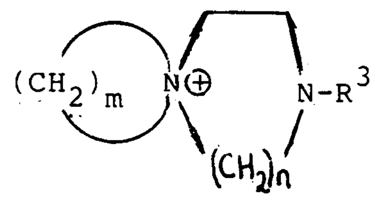

(VII)

(where m, n and $R^3$ are as defined in Claim 1); or

(c) an amine having the formula $H_2N-(CH_2)_m-A^1$ (where m and $A^1$ are as defined above) or a salt thereof is reacted with an acid having the formula $R(CO_2H)_2$ (where R is as defined above)or a reactive derivative thereof to form an imide; or

(d) an amidic acid having the formula

$CO_2H-R-CO-NH-(CH_2)_m-A^1$

(where R, m and $A^1$ are as defined above) or a reactive derivative thereof is cyclised to form an imide; or

(e) an N-substituted maleimide having the formula IX

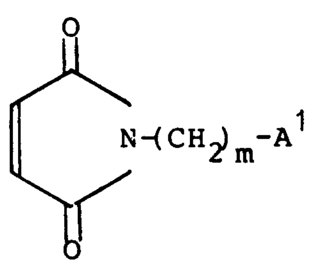

(IX)

(where $A^1$ and m are as defined above) or a salt thereof is subjected to a Diels-Alder addition by reaction with a diene having the formula X, XI or XII

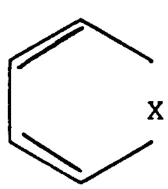

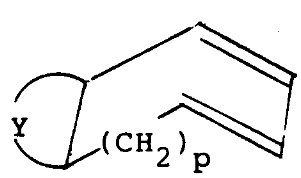

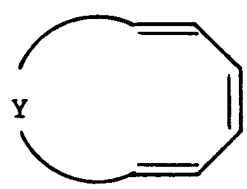

(X) (XI) (XII)

(where X, Y and p are as defined in Claim 1, the diene being other than benzene); or

(f) a compound having the formula

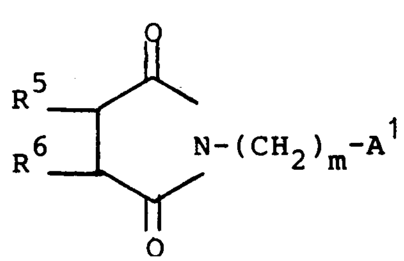

(XVI)

or a salt thereof (where $A^1$ and m are as defined above) and $R^5$ and $R^6$ together represent a group of formula XVII, XVIII or XXI

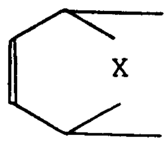
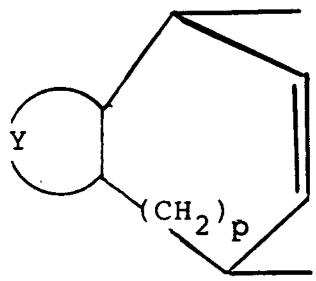
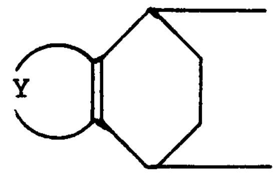

where X, Y and p are as defined in Claim 1 and p is not zero in formula XVIII where Y is $-(CH_2)_o-$ where o is zero) is reduced to convert an ethylenically unsaturated compound into a corresponding saturated compound; or

(g) a compound having formula I as defined and illustrated above is converted into a salt thereof by addition of an acid or a salt of a compound having formula I is converted into a compound having formula I by subtraction of an acid.

**11.** A compound as claimed in any one of Claims 1 to 7 for use as a pharmaceutical

**Claims for the following Contracting States : AT, ES, GR**

**1.** A process for the preparation of a compound having the formula

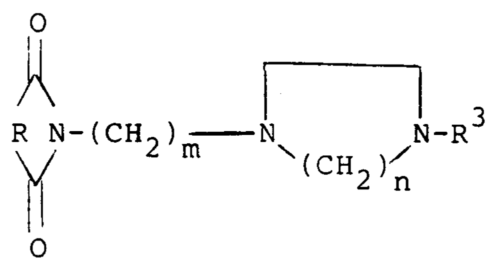

wherein R represents formula III or represents $-CHR^1-CHR^2-$ where $R^1$ and $R^2$ taken together represent formula II or IV

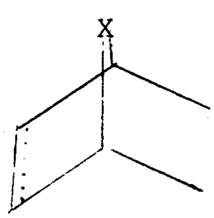
(II)

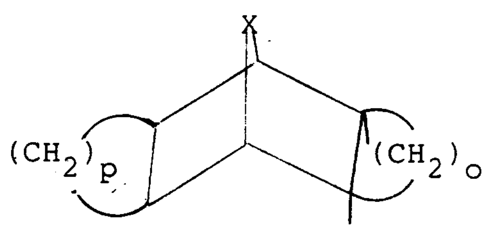
(III)

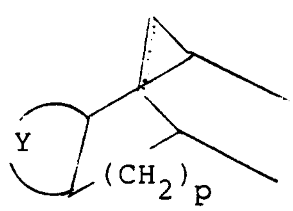
(IV)

$R^3$ is unsubstituted or substituted phenyl, 2-pyridinyl, 2-pyrimidinyl, 2-pyrazinyl, or 3-pyridazinyl; where the substituents are selected from the group $C_1$-$C_6$ alkyl, $C_1$-$C_6$ alkoxy, halo, cyano, nitro and trifluoromethyl with the proviso that when $R^1$ and $R^2$ taken together represent formula II in which X is O, methylene or dimethylene $R^3$ is other than unsubstituted or substituted phenyl, 2-pyridinyl or 2-pyrimidinyl;
Y is $-(CH_2)_o-$ or vinylene;
X is $C_1$-$C_4$ alkylene, vinylene or O;
m is 2 - 4;

n is 1 - 3;
o is 0 - 3;
p is 0 - 4;
..... represents a single or double bond; subject to the proviso that the sum of o and p in formula III and in formula IV where Y is $-(CH_2)_o-$ is not zero;
or a pharmaceutically acceptable salt thereof, in which

(a) a compound having the formula $A^1H$ or a salt thereof (in which $A^1$ is a group having the formula V

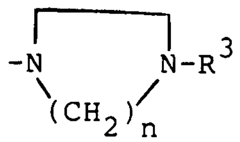

(V)

where $R^3$ and n are as defined above) is N-alkylated to introduce the substituted alkyl group of formula $A^2-(CH_2)_m-$ (in which m is as defined above and $A^2$ is a group of formula VI

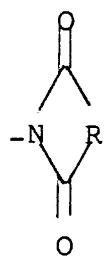

(VI)

where R is as defined above) by reaction with a compound having the formula $A^2-(CH_2)_m-Z^1$ (where $A^2$ and m are as defined above and $Z^1$ is a leaving group) as alkylating agent or by reductive alkylation with an aldehyde having the formula $A^2-(CH_2)_{m-1}-CHO$ (where $A^2$ and m are as defined above); or

(b) an imide having the formula $A^2H$(where $A^2$ is as defined above) or a salt thereof is N-alkylated to introduce the substituted alkyl group of formula $A^1-(CH_2)_m-$ (where $A^1$ and m are as defined above) by reaction with a compound having the formula $A^1-(CH_2)_m-Z^2$ (where $Z^2$ is a leaving group and $A^1$ and m are as defined above) or a compound containing the cation having the formula VII

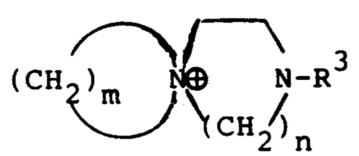

(VII)

(where $R^3$, m and n are as defined above) as alkylating agent; or

(c) an amine having the formula $H_2N-(CH_2)_m-A^1$ or a salt or reactive derivative thereof (where $A^1$ and m are as defined above) is reacted with an acid having the formula $R(CO_2H)_2$ (where R is as defined above) or a reactive derivative thereof to form an imide; or

(d) an amidic acid having the formula

$CO_2H-R-COHN-(CH_2)_m-A^1$ (VIII)

or a reactive derivative thereof is cyclised to form an imide; or

(e) an N-substituted maleimide having the formula IX

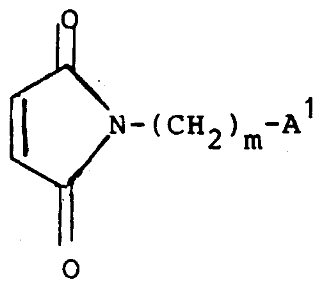

(IX)

(where m and $A^1$ are as defined above) or a salt thereof is subjected to a Diels-Alder addition by reaction with a diene having the formula X, XI or XII

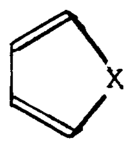

(X)

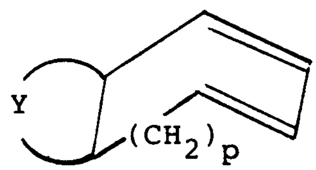

(XI)

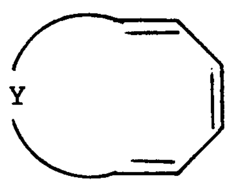

(XII)

(where X, Y and p are as defined in Claim 1, the diene being other than benzene); or
(f) a compound having the formula XVI

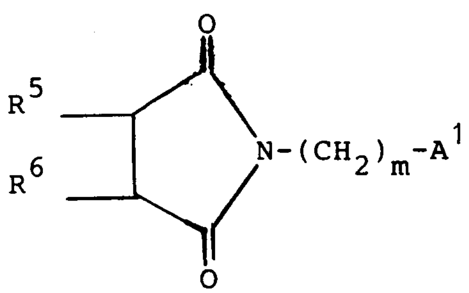

(XVI)

or a salt thereof (where $A^1$ and m are as defined above and $R^5$ and $R^6$ together represent a group having the formula XVII, XVIII or XXI

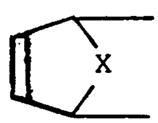

(XVII)

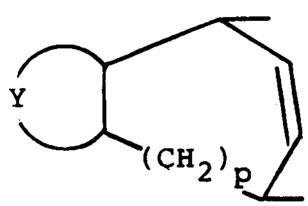

(XVIII)

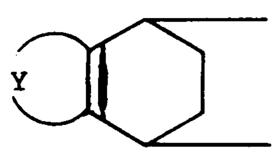

(XXI)

(wherein X, Y and P are as defined in Claim 1 and p is not zero in formula XVIII if Y is $-(CH_2)_o-$ where o is zero) is reduced to convert an ethylenically unsaturated compound into a corresponding saturated compound; or
(g) a compound having formula I is converted into a salt thereof by addition of an acid or a salt of a compound having formula I is converted into a compound having formula I by subtraction of an acid.

2. A process as claimed in Claim 1, wherein $R^3$ is unsubstituted or substituted 2-pyrazinyl or 3-pyridazinyl the substituents being as explained in Claim 1.

3. A process as claimed in Claim 1, wherein R is -$CHR^1$-$CHR^2$- where $R^1$ and $R^2$ together represent formula IV as defined and illustrated in CLaim 1 and o is other than zero.

4. A process for the preparation of a pharmaceutical composition which comprises bringing a compound having the formula I as illustrated and defined in Claim 1 or a pharmaceutically acceptable salt thereof into association or combination with a pharmaceutically acceptable carrier.

5. A process as claimed in Claim 4, wherein $R^3$ is as defined in Claim 2.

6. A process as claimed in Claim 4, wherein R is as defined in Claim 3.

7. A process as claimed in any one of claims 4 to 6, wherein the pharmaceutical composition is prepared in unit dosage form as an antipsychotic agent.

8. A process as claimed in Claim 1 or 4, wherein the compound having formula I is 2-[4-[4-(6-chloro-2-pyrazinyl)-1-piperazinyl]butyl]hexahydro-4,6-ethenocycloprop[f] isoindole-1,3-(2H,3aH)-dione.

9. A process as claimed in Claim 1 or 4 wherein the the compound having formula I is 4,4a,5,5a,6,6a-hexahydro-2-[4-[4-(2-pyrimidinyl)-1-piperazinyl]butyl]-4-6-ethanocycloprop[f]isoindole-1,3-(2H,3aH)-dione.

10. A process as claimed in Claim 1 or 4, wherein the compound having formula I is 3a,4,4a,6a,7,7a-hexahydro-2-[4-[4-(2-pyrimidinyl)-1-piperazinyl]butyl]-4,7-etheno-1H-cyclobut[f]isoindole-1,3-(2H)-dione.

**Patentansprüche**
**Patentansprüche für folgende Vertragsstaaten : BE, CH, DE, FR, IT, LI, LU, NL, SE**

1. Verbindung der Formel

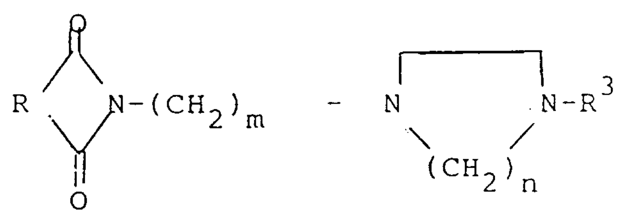

,

worin R Formel (III)

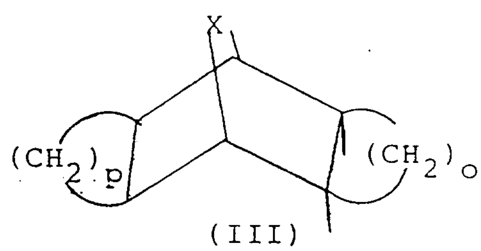

(III)

darstellt oder -$CHR^1$-$CHR^2$- bedeutet, wobei $R^1$ und $R^2$ zusammen Formel (II) oder (IV)

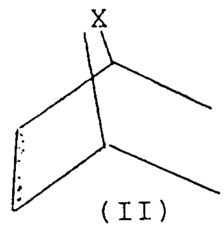

(II)

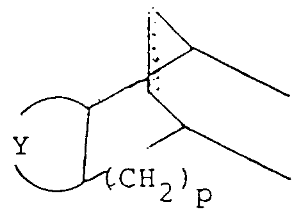

(IV)

darstellen, $R^3$ unsubstituiertes oder substituiertes Phenyl, 2-Pyridinyl, 2-Pyrimidinyl, 2-Pyrazinyl oder 3-Pyridazinyl ist; wobei die Substituenten aus der Gruppe $C_1$-$C_6$-Alkyl, $C_1$-$C_6$-Alkoxy, Halogen, Cyano, Nitro und Trifluormethyl ausgewählt sind, mit der Maßgabe, daß, wenn $R^1$ und $R^2$ zusammen Formel (II) darstellen, worin X die Bedeutung O, Methylen oder Dimethylen hat, $R^3$ von unsubstituiertem oder substituiertem Phenyl, 2-Pyridinyl oder 2-Pyrimidinyl verschieden ist; Y die Bedeutung $-(CH_2)_o-$ oder Vinylen hat; X $C_1$-$C_4$-Alkylen, Vinylen oder O darstellt; m 2 bis 4 ist; n 1 bis 3 ist; o Null bis 3 ist; p Null bis 4 ist; ..... eine Einfach- oder Doppelbindung bedeutet; mit der Maßgabe, daß die Summe von o und p in Formel (III) und in Formel (IV), wenn Y $-(CH_2)_o-$ darstellt, nicht Null ist; oder ein pharmazeutisch annehmbares Salz hievon.

**2.** Verbindung nach Anspruch 1, worin $R^3$ unsubstituiertes oder substituiertes 2-Pyrazinyl oder 3-Pyridazinyl bedeutet, wobei die Substituenten wie in Anspruch 1 definiert sind.

**3.** Verbindung nach Anspruch 1, worin R die Bedeutung $-CHR^1-CHR^2-$ hat, wobei $R^1$ und $R^2$ zusammen Formel (IV) wie in Anspruch 1 definiert und veranschaulicht darstellen und o von Null verschieden ist.

**4.** 2-[4-[4-(6-Chlor-2-pyrazinyl)-1-piperazinyl]-butyl]-hexahydro-4,6-ethenocycloprop[f]isoindol-1,3-(2H,3aH)-dion oder ein pharmazeutisch annehmbares Salz hievon.

**5.** 4,4a,5,5a,6,6a-Hexahydro-2-[4-[4-(2-pyrimidinyl)-1-piperazinyl]-butyl]-4,6-ethenocycloprop[f]isoindol-1,3-(2H,3aH)-dion oder ein pharmazeutisch annehmbares Salz hievon.

**6.** 3a,4,4a,6a,7,7a-Hexahydro-2-[4-[4-(2-pyrimidinyl)-1-piperazinyl]-butyl]-4,7-etheno-1H-cyclobut[f]isoindol-1,3-(2H)-dion oder ein pharmazeutisch annehmbares Salz hievon.

**7.** Verbindung ausgewählt aus 3a,4,4a,6a,7a-Hexahydro-2-[4-[4-(6-Chlor-2-pyrazinyl)-1-piperazinyl]-butyl]-4,7-etheno-1H-cyclobut[f]isoindol-1,3-(2H)-dion, 2-[4-[4-(6-Chlor-2-pyrazinyl)-1-piperazinyl]-butyl]-octahydro-4,7-ethano-1H-cyclobut[f]isoindol-1,3-(2H)-dion, 2-[4-[4-(2-Pyrimidinyl)-1-piperazinyl]-butyl]-octahydro-4,7-ethano-1H-cyclobut[f]isoindol-1,3-(2H)-dion, 2-[4-[4-(2-Pyrimidinyl)-1-piperazinyl]-butyl]-octahydro-4,6-ethanocycloprop[f]isoindol-1,3-(2H,3aH)-dion, 2-[4-[4-(6-Chlor-2-pyrazinyl)-1-piperazinyl]-butyl]-octahydro-4,9-etheno-1H-cycloocta[c]pyrrol-1,3-(2H)-dion, 2-[4-[4-(6-Chlor-3-pyridazinyl)-1-piperazinyl]-butyl]-hexahydro-4,6-ethenocycloprop[f]isoindol-1,3-(2H,3H)-dion, 2-[4-[4-(3-Chlor-2-pyrazinyl)-1-piperazinyl]-butyl]-octahydro-4,7-etheno-1H-cyclobut[f]isoindol-1,3-(2H)-dion, 3a,4,4a,6a,7,7a-Hexahydro-2-[4-[4-(6-chlor-3-pyridazinyl)-1-piperazinyl]-butyl]-4,7-etheno-1H-cyclobut[f]isoindol-1,3-(2H)-dion, 3a,4,4a,6a,7,7a-Hexahydro-2-[4-[4-(3-chlor-2-pyrazinyl)-1-piperazinyl]-butyl]-4,7-etheno-1H-cyclobut[f]isoindol-1,3-(2H)-dion, 3a,4,7,7a-Tetrahydro-2-[4-[4-(6-chlor-2-pyrazinyl)-1-piperazinyl]-butyl]-4,7-methano-1H-isoindol, 2-[4-[4-(6-Chlor-2-pyrazinyl)-1-piperazinyl]-butyl]-octahydro-4,9-ethano-1H-cycloocta[c]pyrrol-1,3-dion, 2-[4-[4-(6-Chlor-2-pyrazinyl)-1-piperazinyl]-butyl]-octahydro-4,7-etheno-1H-cyclobut[f]isoindol-1,3-(2H)-dion, 2-[4-[4-(2-Pyrimidinyl)-1-piperazinyl]-butyl]-octahydro-4,7-etheno-1H-cyclobut[f]isoindol-1,3-(2H)-dion, 2-[4-[4-(6-Chlor-3-pyridazinyl)-1-pipera zinyl]-butyl]-3a,4,7,7a-tetrahydro-4,7-methano-1H-isoindol-1,3-(2H)-dion, 2-[4-[4-(3-Chlor-2-pyrazinyl)-1-piperazinyl]-butyl]-3a,4,7,7a-tetrahydro-4,7-methano-1H-isoindol-1,3-(2H)-dion, 2-[4-[4-(3-Chlor-2-pyrazinyl)-1-piperazinyl]-butyl]-hexahydro-4,6-ethenocycloprop[f]isoindol-1,3-(2H,3aH)-dion, 2-[4-[4-(3-Chlor-2-pyrazinyl)-1-piperazinyl]-butyl]-octahydro-4,9-etheno-1H-cycloocta[c]pyrrol-1,3-(2H)-dion, 4,5,6,7,8,8a-Hexahydro-2-[4-[4-(2-pyrimidinyl)-1-piperazinyl]-butyl]-4,8-ethenocyclohepta[c]pyrrol-1,3-(2H,3aH)-dion, 2-[4-[4-(6-Chlor-2-pyrazinyl)-1-piperazinyl]-butyl]-4,5,6,7,8,8a-hexahydro-4,8-ethenocyclohepta[c]pyrrol-1,3-(2H,3aH)-dion, 3a,4,4a,6a,7,7a-Hexahydro-2-[4-[4-(2-pyrazinyl)-1-piperazinyl]-butyl]-4,7-etheno-1H-cyclobut[f]isoindol-1,3-(2H)-dion, 4,4a,5,5a,6,6 a-Hexahydro-2-[4-[4-(2-pyrazinyl)-1-piperazinyl]-butyl]-4,6-ethenocycloprop[f]isoindol-1,3-(2H,3aH)-dion, 2-[4-[4-(6-Chlor-2-pyrazinyl)-1-piperazinyl]-butyl]-hexahydro-4,6-ethanocycloprop[f]isoindol-1,3-(2H,3aH)-dion, 2-[4-[4-(3-Chlor-2-pyrazinyl)-1-piperazinyl]-butyl]-hexahydro-4,6-ethanocycloprop[f]isoindol-1,3-(2H,3aH)-dion, 3a,4,4a,5,6,6a,7,7a-Octahydro-2-[4-[4-(2-pyrazinyl)-piperazinyl]-butyl]-4,7-etheno-1H-cyclobut[f]isoindol-1,3-(2H)-dion, 3a,4,4a,6a,7,7a-Hexahydro-2-[4-[4-(5-trifluormethyl-2-pyridinyl)-1-piperazinyl]-butyl]-4,7-etheno-1H-cyclobut[f]isoindol-1,3-(2H)-dion, 4,5,6,7,8,8a-Hexahydro-2-[4-[4-(2-pyrimidinyl)-1-piperazinyl]-butyl]-4,8-ethanocyclohepta-[c]pyrrol-1,3-(2H,3aH)-dion und deren pharmazeutisch annehmbare Salze.

**8.** Pharmazeutische Zusammensetzung umfassend eine Verbindung nach einem der Ansprüche 1 bis 7 in Vereinigung oder Kombination mit einem pharmazeutisch annehmbaren Träger.

**9.** Pharmazeutische Zusammensetzung nach Anspruch 8 in Einheitsdosierungsform als antipsychotisches Mittel.

**10.** Verfahren zur Herstellung einer Verbindung der Formel $A^1$-$(CH_2)_m$-$A^2$, worin $A^1$ eine Gruppe der Formel (V)

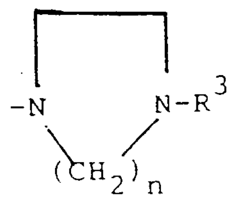

(V)

bedeutet, wobei $R^3$ und n wie in Anspruch 1 definiert sind, m wie in Anspruch 1 definiert ist und $A^2$ Formel (VI)

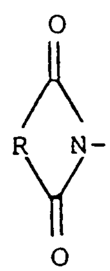

(VI)

darstellt, wobei R wie in Anspruch 1 definiert ist, oder eines Salzes hievon, bei welchem

(a) eine Verbindung der Formel $A^1$-H (worin $A^1$ wie oben definiert ist) oder ein Salz hievon N-alkyliert wird, wobei eine substituierte Alkylgruppe durch Umsetzen mit einer Verbindung der Formel $A^2$-$(CH_2)_m$-$Z^1$ als Alkylierungsmittel (worin $A^2$ und m wie oben definiert sind und $Z^1$ eine Abgangsgruppe ist) oder durch reduktive Alkylierung mit einem Aldehyd der Formel $A^2$-$(CH_2)_{m-1}$-CHO (worin $A^2$ und m wie oben definiert sind) eingeführt wird; oder

(b) ein Imid der Formel $A^2$H (worin $A^2$ wie oben definiert ist) oder ein Salz hievon N-alkyliert wird, wobei eine substituierte Alkylgruppe durch Umsetzen mit einer Verbindung der Formel $A^1$-$(CH_2)_m$-$Z^2$, worin $A^1$ und m wie oben definiert sind und $Z^2$ eine Abgangsgruppe ist, oder mit einer Verbindung enthaltend das Kation der Formel (VII)

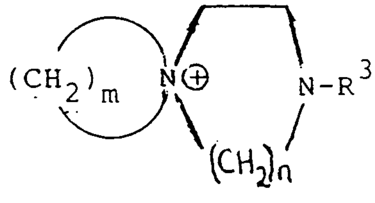

(VII)

(worin m, n und $R^3$ wie in Anspruch 1 definiert sind) eingeführt wird; oder
(c) ein Amin der Formel $H_2N-(CH_2)_m-A^1$ (worin m und $A^1$ wie oben definiert sind) oder ein Salz hievon mit einer Säure der Formel $R(CO_2H)_2$ (worin R wie oben definiert ist) oder einem reaktiven Derivat hievon umgesetzt wird, wobei ein Imid gebildet wird; oder
(d) ein Säureamid der Formel

$CO_2H-R-CO-NH-(CH_2)_m-A^1$

(worin R, m und $A^1$ wie oben definiert sind) oder ein reaktives Derivat hievon cyclisiert wird, wobei ein Imid gebildet wird; oder
(e) ein N-substituiertes Maleimid der Formel (IX)

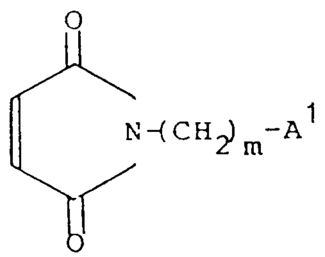

(IX)

(worin $A^1$ und m wie oben definiert sind) oder ein Salz hievon einer Diels-Alder-Addition durch Umsetzen mit einem Dien der Formel (X), (XI) oder (XII)

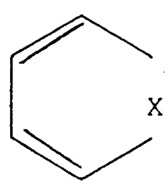

(X)

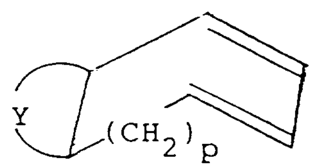

(XI)

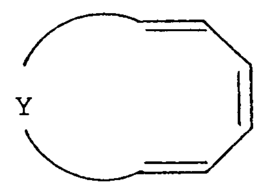

(XII)

(worin X, Y und p wie in Anspruch 1 definiert sind, wobei das Dien von Benzol verschieden ist) unterworfen wird; oder
(f) eine Verbindung der Formel (XVI)

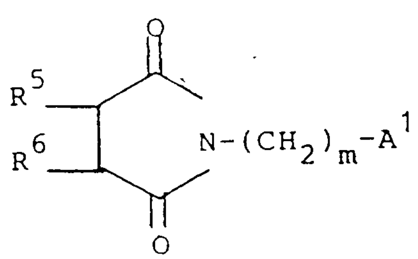

(XVI)

oder ein Salz hievon (worin $A^1$ und m wie oben definiert sind und $R^5$ und $R^6$ zusammen eine Gruppe der Formel (XVII), (XVIII) oder (XXI)

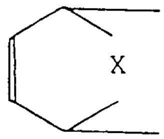

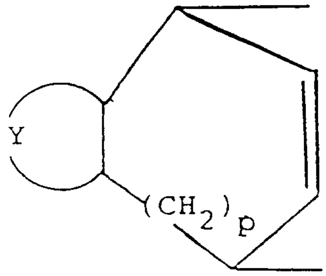

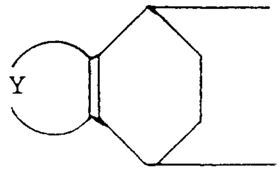

darstellen, worin X, Y und p wie in Anspruch 1 definiert sind und p in Formel (XVIII) nicht Null ist, wenn Y die Bedeutung $-(CH_2)_o-$ hat, wobei o Null ist) reduziert wird, wobei eine ethylenisch ungesättigte Verbindung in eine entsprechende gesättigte Verbindung übergeführt wird; oder
(g) eine Verbindung der Formel (I) wie oben definiert und veranschaulicht in ein Salz hievon durch Addition einer Säure übergeführt wird oder ein Salz einer Verbindung der Formel (I) in eine Verbindung der Formel (I) durch Subtraktion einer Säure übergeführt wird.

**11.** Verbindung nach einem der Ansprüche 1 bis 7 zur Verwendung als Pharmazeutikum.

**Patentansprüche für folgende Vertragsstaaten : AT, ES, GR**

**1.** Verfahren zur Herstellung einer Verbindung der Formel worin R Formel (III)

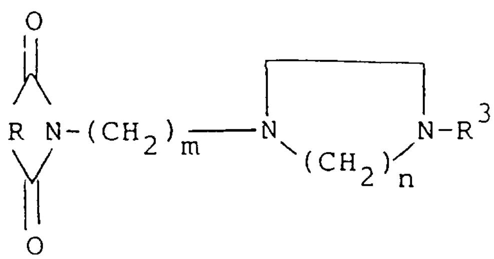

,

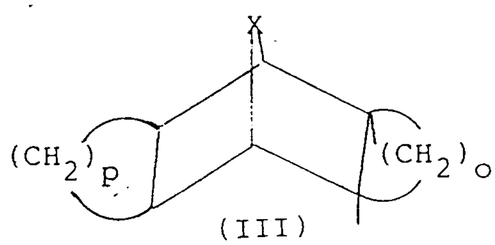

(III)

darstellt oder $-CHR^1-CHR^2-$ bedeutet, wobei $R^1$ und $R^2$ zusammen Formel (II) oder (IV)

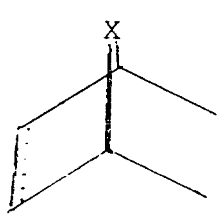

(II)

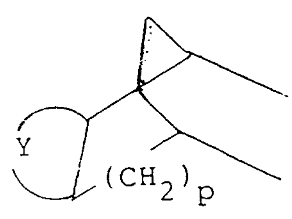

(IV)

darstellen, $R^3$ unsubstituiertes oder substituiertes Phenyl, 2-Pyridinyl, 2-Pyrimidinyl, 2-Pyrazinyl oder 3-Pyridazinyl ist; wobei die Substituenten aus der Gruppe $C_1$-$C_6$-Alkyl, $C_1$-$C_6$-Alkoxy, Halogen, Cyano,

Nitro und Trifluormethyl ausgewählt sind, mit der Maßgabe, daß, wenn $R^1$ und $R^2$ zusammen Formel (II) darstellen, worin X die Bedeutung O, Methylen oder Dimethylen hat, $R^3$ von unsubstituiertem oder substituiertem Phenyl, 2-Pyridinyl oder 2-Pyrimidinyl verschieden ist; Y die Bedeutung $-(CH_2)_o-$ oder Vinylen hat; X $C_1$-$C_4$-Alkylen, Vinylen oder O darstellt; m 2 bis 4 ist; n 1 bis 3 ist; o Null bis 3 ist; p Null bis 4 ist; ..... eine Einfach- oder Doppelbindung bedeutet; mit der Maßgabe, daß die Summe von o und p in Formel (III) und in Formel (IV), wenn Y $-(CH_2)_o-$ darstellt, nicht Null ist; oder ein pharmazeutisch annehmbares Salz hievon, bei welchem

(a) eine Verbindung der Formel $A^1$-H oder ein Salz hievon (worin $A^1$ eine Gruppe der Formel (V)

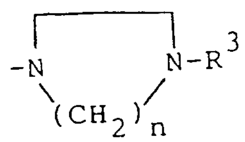

(V)

bedeutet, wobei $R^3$ und n wie in Anspruch 1 definiert sind) N-alkyliert wird, wobei die substituierte Alkylgruppe der Formel $A^2$-$(CH_2)_m$- (worin m wie oben definiert ist und $A^2$ eine Gruppe der Formel (VI)

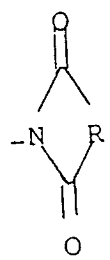

(VI)

bedeutet, wobei R wie oben definiert ist) durch Umsetzen mit einer Verbindung der Formel $A^2$-$(CH_2)_m$-$Z^1$ (worin $A^2$ und m wie oben definiert sind und $Z^1$ eine Abgangsgruppe ist) als Alkylierungsmittel oder durch reduktive Alkylierung mit einem Aldehyd der Formel $A^2$-$(CH_2)_{m-1}$-CHO (worin $A^2$ und m wie oben definiert sind) eingeführt wird; oder

(b) ein Imid der Formel $A^2H$ (worin $A^2$ wie oben definiert ist) oder ein Salz hievon N-alkyliert wird, wobei die substituierte Alkylgruppe der Formel $A^1$-$(CH_2)_m$- (worin $A^1$ und m wie oben definiert sind) durch Umsetzen mit einer Verbindung der Formel $A^1$-$(CH_2)_m$-$Z^2$ (worin $Z^2$ eine Abgangsgruppe ist und $A^1$ und m wie oben definiert sind) oder mit einer Verbindung enthaltend das Kation der Formel (VII)

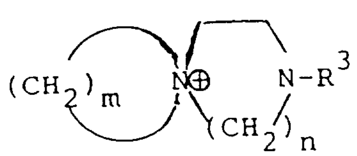

(VII)

(worin $R^3$, m und n wie oben definiert sind) als Alkylierungsmittel eingeführt wird; oder

(c) ein Amin der Formel $H_2N$-$(CH_2)_m$-$A^1$ oder ein Salz oder reaktives Derivat hievon (worin $A^1$ und m wie oben definiert sind) mit einer Säure der Formel $R(CO_2H)_2$ (worin R wie oben definiert ist) oder einem reaktiven Derivat hievon umgesetzt wird, wobei ein Imid gebildet wird; oder

(d) ein Säureamid der Formel

$CO_2H$-R-COHN-$(CH_2)_m$-$A^1$ (VIII)

oder ein reaktives Derivat hievon cyclisiert wird, wobei ein Imid gebildet wird; oder

(e) ein N-substituiertes Maleimid der Formel (IX)

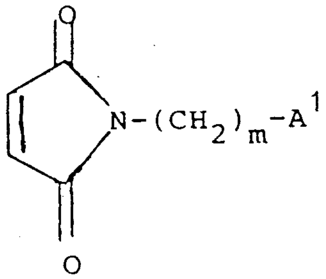

(IX)

(worin m und $A^1$ wie oben definiert sind) oder ein Salz hievon einer Diels-Alder-Addition durch Umsetzen mit einem Dien der Formel (X), (XI) oder (XII)

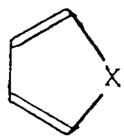

(X)

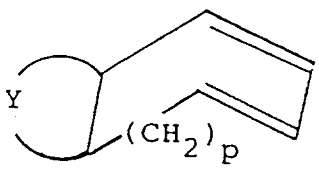

(XI)

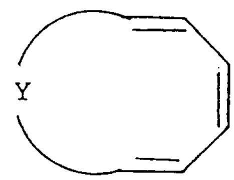

(XII)

(worin X, Y und p wie in Anspruch 1 definiert sind, wobei das Dien von Benzol verschieden ist) unterworfen wird; oder
(f) eine Verbindung der Formel (XVI)

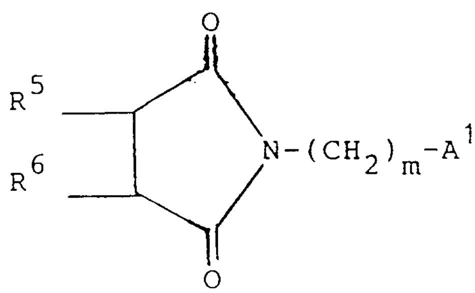

(XVI)

oder ein Salz hievon (worin $A^1$ und m wie oben definiert sind und $R^5$ und $R^6$ zusammen eine Gruppe der Formel (XVII), (XVIII) oder (XXI)

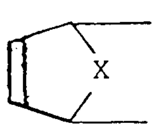

(XVII)

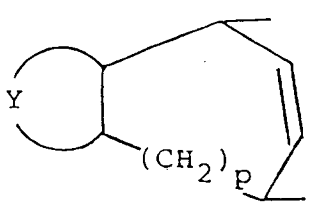

(XVIII)

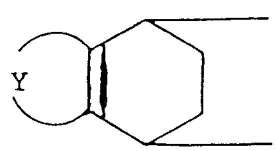

(XXI)

darstellen, worin X, Y und p wie in Anspruch 1 definiert sind und p in Formel (XVIII) nicht Null ist, wenn Y die Bedeutung $-(CH_2)_o-$ hat, wobei o Null ist) reduziert wird, wobei eine ethylenisch ungesättigte Verbindung in eine entsprechende gesättigte Verbindung übergeführt wird; oder
(g) eine Verbindung der Formel (I) in ein Salz hievon durch Addition einer Säure übergeführt wird oder ein Salz einer Verbindung der Formel (I) in eine Verbindung der Formel (I) durch Subtraktion

einer Säure übergeführt wird.

2. Verbindung nach Anspruch 1, worin $R^3$ unsubstituiertes oder substituiertes 2-Pyrazinyl oder 3-Pyridazinyl bedeutet, wobei die Substituenten wie in Anspruch 1 definiert sind.

3. Verbindung nach Anspruch 1, worin R die Bedeutung $-CHR^1-CHR^2-$ hat, wobei $R^1$ und $R^2$ zusammen Formel (IV) wie in Anspruch 1 definiert und veranschaulicht darstellen und o von Null verschieden ist.

4. Verfahren zur Herstellung einer pharmazeutischen Zusammensetzung, welches das Vereinigen oder Kombinieren einer Verbindung der Formel (I) wie in Anspruch 1 veranschaulicht und definiert oder eines pharmazeutisch annehmbaren Salzes hievon mit einem pharmazeutisch annehmbaren Träger umfaßt.

5. Verfahren nach Anspruch 4, bei welchem $R^3$ wie in Anspruch 2 definiert ist.

6. Verfahren nach Anspruch 4, bei welchem R wie in Anspruch 3 definiert ist.

7. Verfahren nach einem der Ansprüche 4 bis 6, bei welchem die pharmazeutische Zusammensetzung in Einheitsdosierungsform als antipsychotisches Mittel hergestellt wird.

8. Verfahren nach Anspruch 1 oder 4, bei welchem die Verbindung der Formel (I) 2-[4-[4-(6-Chlor-2-pyrazinyl)-1-piperazinyl]-butyl]-hexahydro-4,6-ethenocycloprop[f]isoindol-1,3-(2H,3aH)-dion ist.

9. Verfahren nach Anspruch 1 oder 4, bei welchem die Verbindung der Formel (I) 4,4a,5,5a,6,6a-Hexahydro-2-[4-[4-(2-pyrimidinyl)-1-piperazinyl]-butyl]-4,6-ethanocycloprop[f]isoindol-1,3-(2H,3aH)-dion ist.

10. Verfahren nach Anspruch 1 oder 4, bei welchem die Verbindung der Formel (I) 3a,4,4a,6a,7,7a-Hexahydro-2-[4-[4-(2-pyrimidinyl)-1-piperazinyl]-butyl]-4,7-etheno-1H-cyclobut[f]isoindol-1,3-(2H)-dionist.

**Revendications**
**Revendications pour les Etats contractants suivants : BE, CH, DE, FR, IT, LI, LU, NL, SE**

1. Composé de formule :

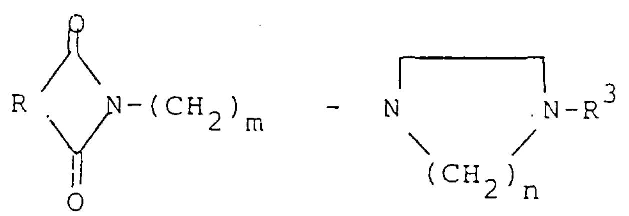

(I)

où R représente la formule III ou $-CHR^1-CHR^2-$ où $R^1$ et $R^2$ pris ensemble représentent la formule II ou IV :

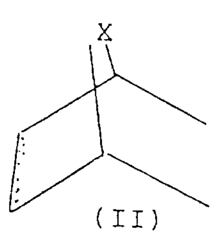

(II)

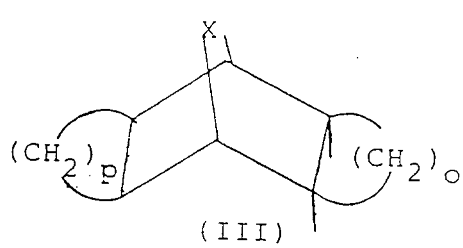

(III)

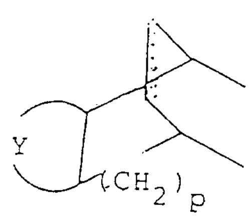

(IV)

$R^3$ est phényle, 2-pyridinyle, 2-pyrimidinyle, 2-pyrazinyle ou 3-pyridazinyle non substitué ou substitué,

dont les substituants sont choisis dans la classe $C_1$-$C_6$-alcoyle,$C_1$-$C_6$-alcoxy, halo, cyano, nitro et trifluorométhyle, avec la restriction que lorsque $R^1$ et $R^2$ pris ensemble représentent la formule II où X est O, méthylène ou diméthylène, $R^3$ est autre que phényle, 2-pyridinyle ou 2-pyrimidinyle non substitué ou substitué;

Y est $-(CH_2)_o-$ ou vinylène;

X est $c_1$-$c_6$-alcoylène, vinylène ou O;

m est 2-4;

n est 1-3;

o est 0-3;

p est 0-4;

...... représente une liaison simple ou double;

avec la restriction que la somme de o et p dans la formule III et dans la formule IV où Y est $-(CH_2)_o-$ n'est pas zéro;

ou un sel pharmaceutiquement acceptable de celui-ci.

**2.** Composé suivant la revendication 1, dans lequel $R^3$ est 2-pyrazinyle ou 3-pyridazinyle non substitué ou substitué, les substituants étant tels que définis dans la revendication 1.

**3.** Composé suivant la revendication 1, dans lequel R représente $-CHR^1-CHR^2-$ où $R^1$ et $R^2$ représentent ensemble la formule IV telle que définie et illustrée dans la revendication 1 et o est autre que zéro.

**4.** La 2-[4-[4-(6-chloro-2-pyrazinyl)-1-pipérazinyl]butyl]hexahydro-4,6-éthénocycloprop[f]isoindole-1,3-(2H,3aH)-dione ou un sel pharmaceutiquement acceptable de celle-ci.

**5.** La 4,4a,5,5a,6,6a-hexahydro-2-[4-[4-(2-pyrimidinyl)-1-pipérazinyl]butyl]-4,6-éthénocycloprop[f]isoindole-1,3-(2H,3aH)-dione ou un sel pharmaceutiquement acceptable de celle-ci.

**6.** La 3a,4,4a,6a,7,7a-hexahydro-2-[4-[4-(2-pyrimidinyl)-1-pipérazinyl]butyl]-4,7-éthéno-1H-cyclobut[f]-isoindole-1,3-(2H)-dione ou un sel pharmaceutiquement acceptable de celle-ci.

**7.** Composé choisi parmi la 3a,4,4a,6a,7,7a-hexahydro-2-[4-[4-(6-chloro-2-pyrazinyl)-1-pipérazinyl]butyl]-4,7-éthéno-1H-cyclobut[f]isoindole-1,3(2H)-dione,la 2-[4-[4-(6-chloro-2-pyrazinyl)-1-pipérazinyl]butyl]-octahydro-4,7-éthano-1H-cyclobut[f]isoindole-1,3-(2H)-dione, la 2-[4-[4-(2-pyrimidinyl)-1-pipérazinyl]-butyl]octahydro-4,7-éthano-1H-cyclobut[f]isoindole-1,3-(2H)dione, la 2-[4-[4-(2-pyrimidinyl)-1-pipérazinyl]butyl]octahydro-4,6-éthanocycloprop[f]isoindole-1,3-(2H,3aH)-dione, la 2-[4-[4-(6-chloro-2-pyrazinyl)-1-pipérazinyl]butyl]octahydro-4,9-éthéno-1H-cycloocta[c]pyrrole-1,3-(2H -dione, la 2-[4-[4-(6-chloro-3-pyridazinyl)-1-pipérazinyl]butyl]hexahydro-4,6-éthénocycloprop[f]isoindole-1,3-(2H,3H)-dione, la 2-[4-[4-(3-chloro-2-pyrazinyl)-1-pipérazinyl]butyl]octahydro-4,7-éthéno-1H-cyclobut[f]isoindole-1,3-(2H)-dione, la 3a,4,4a,6a,7,7a-hexahydro-2-[4-[4-(6-chloro-3-pyridazinyl]-1-pipérazinyl]butyl]-4,7-éthéno-1H-cyclobut[f] isoindole-1,3-(2H)-dione, la 3a,4,4a,6a,7,7a-hexahydro-2-[4-[4-(3-chloro-2-pyrazinyl)-1-pipérazinyl]-butyl]-4,7-éthéno-1H-cyclobut[f]-isoindole-1,3-(2H)-dione, la 3a,4,7,7a-tétrahydro-2-[4-[4-(6-chloro-2-pyrazinyl)-1-pipérazinyl]butyl] -4,7-méthano-1H-isoindole-1,3-(2H)-dione, la 2-[4-[4-(6-chloro-2-pyrazinyl)-1-pipérazinyl]butyl]octahydro-4,9-éthano-1H-cycloocta[c]pyrrole-1,3-dione, la 2-[4-[4-(6-chloro-2-pyrazinyl)-1-pipérazinyl]butyl]octahydro-4,7-éthéno-1H-cyclobut[f]isoindole-1,3-(2H)-dione, la 2-[4-[4-(2-pyrimidinyl)-1-pipérazinyl]butyl)octahydro-4,7-éthéno-1H-cyclobut[f]isoindole-1,3-(2H)dione, la 2-[4-[4-(6-chloro-3-pyridazinyl)-1-pipérazinyl]butyl]-3a,4,7,7a-tétrahydro-4,7-méthano-1H-isoindole-1,3-(2H)-dione, la 2-[4-[4-(3-chloro-2-pyrazinyl)-1-pipérazinyl]butyl]-3a,4,7,7a-tétrahydro-4,7-méthano-1H-isoindole-1,3-(2H)-di one, la 2-[4-[4-(3-chloro-2-pyrazinyl)-1-pipérazinyl]butyl]hexahydro-4,6-éthénocycloprop[f]isoindole-1,3-(2H,3aH)-dione, la 2-[4-[4-(3-chloro-2-pyrazinyl)-1-pipérazinyl]butyl]-octahydro-4,9-éthéno-1H-cycloocta[c]pyrrole-1,3-(2H)-dione, la 4,5,6,7,8,8a-hexahydro-2-[4-[4-(2-pyrimidinyl)-1-pipérazinyl]butyl]-4,8-éthénocyclohepta[c]pyrrole-1,3-(2H,3aH)-dione, la 2-[ 4-[4-(6-chloro-2-pyrazinyl)-1-pipérazinyl]butyl]-4,5,6,7,8,8a-hexahydro-4,8-éthénocyclo-hepta[c]pyrrole-1,3(2H,3aH)-dione, la 3a,4,4a,6a,7,7a-hexahydro-[2-[4-[4-(2(pyrazinyl)-1-pipérazinyl]butyl]-4,7-éthéno-1H-cyclobut[f]-isoindole-1,3-(2H)-dione, la 4,4a,5,5a,6,6a-hexahydro-[2-[4-[4-(2(pyrazinyl)-1-pipérazinyl]butyl]-4,6-éthénocycloprop[f]isoindole-1,3-(2H,3aH )-dione, la 2-[4-[4-(6-chloro-2-pyrazinyl)-1-pipérazinyl]butyl]-hexahydro-4,6-éthanocycloprop[f]-isoindole-1,3(2H,3aH)-dione, la 2-[4-[4-(3-chloro-2-pyrazinyl)-1-pipérazinyl]butyl]hexahydro-4,6-éthanocycloprop[f]isoindole-1,3-(2H,3aH)-dione, la 3a,4,4a,5,6,6a,7,7a-octahydro[2-[4-[4-(2-(pyrazinyl)-pipérazinyl]butyl]-4,7-éthéno-1H-cyclobut[f]isoindole-1,3(2H)-dione, la

3a,4,4a,6a,7,7a-hexahydro-[2-[4-[4-(5-trifluorométhyl-2-pyridinyl)-1-pipérazinyl]butyl]-4,7-éthéno-1H-cyclobut[f]isoindole-1,3-(2H)-dione, la 4,5,6,7,8,8a-hexahydro-2-[4-[4-(2-pyrimidinyl)-1-pipérazinyl]butyl]-4,8-éthano-cyclohepta[c]pyrrole-1,3-(2H,3aH)-dione et leurs sels pharmaceutiquement acceptables.

**8.** Composition pharmaceutique comprenant un composé suivant l'une quelconque des revendications 1 à 7 en association ou combinaison avec un excipient pharmaceutiquement acceptable.

**9.** Composition pharmaceutique suivant la revendication 8 sous forme dosée unitaire comme agent antipsychotique.

**10.** Procédé de préparation d'un composé de formule :

$A^1-(CH_2)_m-A^2$

où $A^1$ représente un radical de formule V :

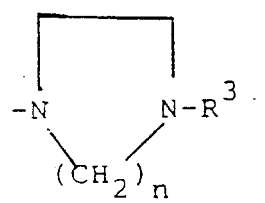

(V)

où $R^3$ et n sont tels que définis dans la revendication 1, m est tel que défini dans la revendication 1 et $A^2$ représente la formule VI :

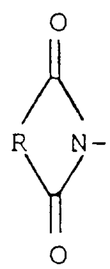

(VI)

où R est tel que défini dans la revendication 1,
ou d'un sel de celui-ci dans lequel :

(a) un composé de formule $A^1$-H (où $A^1$ est tel que défini ci-dessus) ou un sel de celui-ci est N-alcoylé pour introduire un radical alcoyle substitué par réaction avec un composé de formule $A^2-(CH_2)_m-Z^1$ comme agent d'alcoylation (où $A^2$ et m sont tels que définis ci-dessus et $Z^1$ est un radical partant) ou par alcoylation réductrice avec un aldéhyde de formule $A^2-(CH_2)_{m-1}-CHO$ (où $A^2$ et m sont tels que définis ci-dessus); ou

(b) un imide de formule $A^2H$ (où $A^2$ est tel que défini ci-dessus) ou un sel de celui-ci est N-alcoylé pour introduire un radical alcoyle substitué par réaction avec un composé de formule $A^1-(CH_2)_m-Z^2$ où $A^1$ et m sont tels que définis ci-dessus et $Z^2$ est un radical partant ou avec un composé contenant le cation de formule VII :

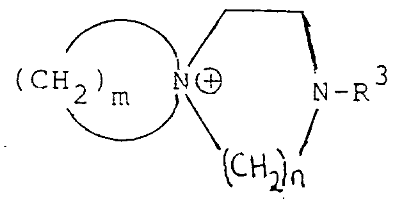

(VII)

(où m, n et $R^3$ sont tels que définis dans la revendication 1); ou

(c) une amine de formule $H_2N-(CH_2)_m-A^1$ (où m et $A^1$ sont tels que définis ci-dessus) ou un sel de celle-ci est mis à réagir avec un acide de formule $R(CO_2H)_2$ (où R est tel que défini ci-dessus) ou un dérivé réactif de celui-ci pour former un imide; ou

(d) un acide amidique de formule :

$CO_2H-R-CO-NH-(CH_2)_m-A^1$

(où R, m et $A^1$ sont tels que définis ci-dessus) ou un dérivé réactif de celui-ci est cyclisé pour former un imide; ou

(e) un maléimide N-substitué de formule IX :

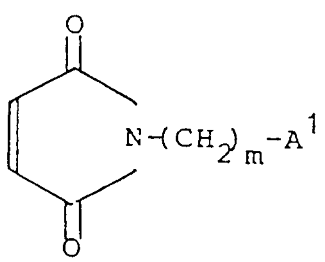

(IX)

(où $A^1$ et m sont tels que définis ci-dessus) ou un sel de celui-ci est soumis à une addition de Diels-Alder par réaction avec un diène de formule X, XI ou XII :

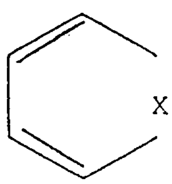

(X)

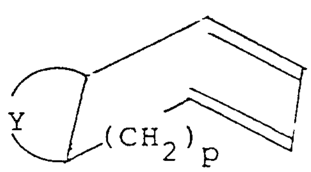

(XI)

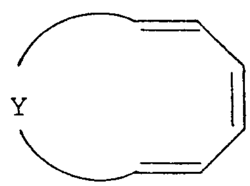

(XII)

(où X, Y et p sont tels que définis dans la revendication 1, le diène étant autre que le benzène); ou

(f) un composé de formule :

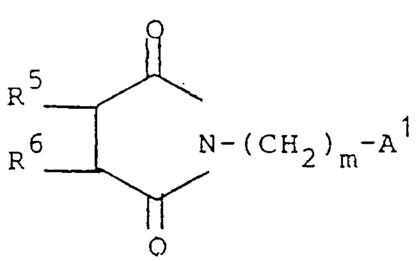

(XVI)

ou un sel de celui-ci (où $A^1$ et m sont tels que définis ci-dessus) et $R^5$ et $R^6$ représentent ensemble un radical de formule XVII, XVIII ou XXI :

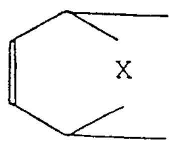 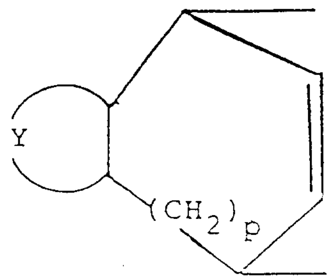 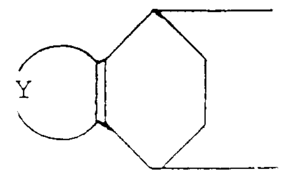

où X, Y et p sont tels que définis dans la revendication 1 et p n'est pas zéro dans la formule XVIII lorsque Y est $-(CH_2)_o-$ où o est zéro) est réduit pour convertir un composé éthyléniquement insaturé en un composé saturé correspondant; ou

(g) un composé de formule I tel que défini et illustré ci-dessus est converti en un sel de celui-ci par addition d'un acide ou un sel d'un composé de formule I converti en un composé de formule I par soustraction d'un acide.

**11.** Composé suivant l'une quelconque des revendications 1 à 7 à utiliser comme agent pharmaceutique.

**Revendications pour les Etats contractants suivants : AT, ES, GR**

**1.** Procédé de préparation d'un composé de formule :

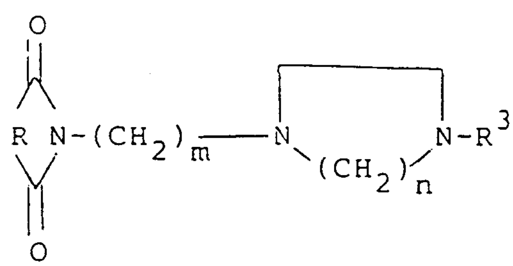

où R représente la formule III ou $-CHR^1-CHR^2-$ où $R^1$ et $R^2$ pris ensemble représentent la formule II ou IV

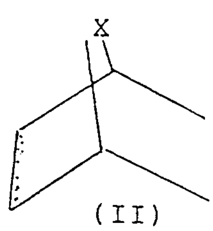
(II)

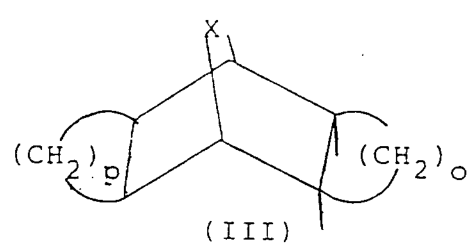
(III)

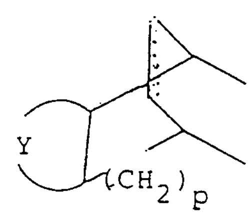
(IV)

$R^3$ est phényle, 2-pyridinyle, 2-pyrimidinyle, 2-pyrazinyle ou 3-pyridazinyle non substitué ou substitué, dont les substituants sont choisis dans la classe $C_1$-$C_6$-alcoyle,$C_1$-$C_6$-alcoxy, halo, cyano, nitro et trifluorométhyle, avec la restriction que lorsque $R^1$ et $R^2$ pris ensemble représentent la formule II où X est O, méthylène ou diméthylène, $R^3$ est autre que phényle, 2-pyridinyle ou 2-pyrimidinyle non substitué ou substitué;

Y est $-(CH_2)_o-$ ou vinylène;

X est $c_1$-$c_6$-alcoylène, vinylène ou O;

m est 2-4;
n est 1-3;
o est 0-3;
p est 0-4;
...... représente une liaison simple ou double;
avec la restriction que la somme de o et p dans la formule III et dans la formule IV où Y est $-(CH_2)_o-$ n'est pas zéro;

ou d'un sel pharmaceutiquement acceptable de celui-ci, dans lequel,

(a) un composé de formule $A^1$-H ou un sel de celui-ci (où $A^1$ est un radical de formule V

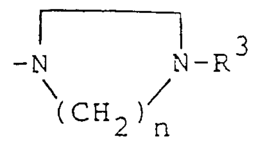

(V)

où $R^3$ et n sont tels que définis ci-dessus) est N-alcoylé pour introduire le radical alcoyle substitué de formule $A^2-(CH_2)_m-$ (où m est tel que défini ci-dessus et $A^2$ est un radical de formule VI

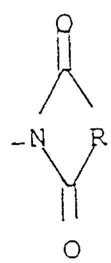

(VI)

où R est tel que défini ci-dessus) par réaction avec un composé de formule $A^2-(CH_2)_m-Z^1$ comme agent d'alcoylation (où $A^2$ et m sont tels que définis ci-dessus et $Z^1$ est un radical partant) ou par alcoylation réductrice avec un aldéhyde de formule $A^2-(CH_2)_{m-1}-CHO$ (où $A^2$ et m sont tels que définis ci-dessus); ou

(b) un imide de formule $A^2H$ (où $A^2$ est tel que défini ci-dessus) ou un sel de celui-ci est N-alcoylé pour introduire un radical alcoyle substitué de formule $A^1-(CH^2)_m-$ (où $A^1$ et m sont tels que définis ci-dessus) par réaction avec un composé de formule $A^1-(CH_2)_m-Z^2$ (où $A^1$ et m sont tels que définis ci-dessus et $Z^2$ est un radical partant) ou avec un composé contenant le cation de formule VII :

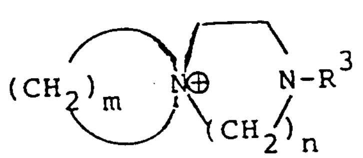

(VII)

(où $R^3$ m et n sont tels que définis ci-dessus) comme agent d'alcoylation; ou

(c) une amine de formule $H_2N-(CH_2)_m-A^1$ ou un sel ou dérivé réactif de celle-ci (où $A^1$ et m sont tels que définis ci-dessus) est mis à réagir avec un acide de formule $R(CO_2H)_2$ (où R est tel que défini ci-dessus) ou un dérivé réactif de celui-ci pour former un imide; ou

(d) un acide amidique de formule :

$CO_2H-R-CO-NH-(CH_2)_m-A^1$ (VIII)

ou un dérivé réactif de celui-ci est cyclisé pour former un imide; ou

(e) un maléimide N-substitué de formule IX :

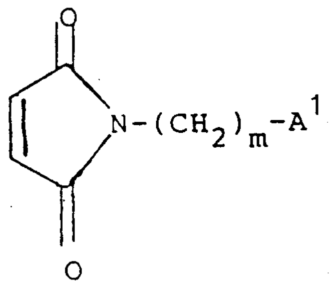

(IX)

(où $A^1$ et m sont tels que définis ci-dessus) ou un sel de celui-ci est soumis à une addition de Diels-Alder par réaction avec un diène de formule X, XI ou XII :

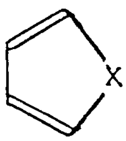

(X)

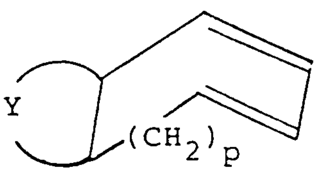

(XI)

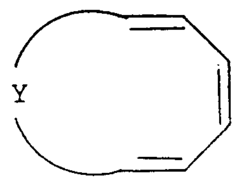

(XII)

(où X, Y et p sont tels que définis dans la revendication 1, le diène étant autre que le benzène); ou
(f) un composé de formule XVI

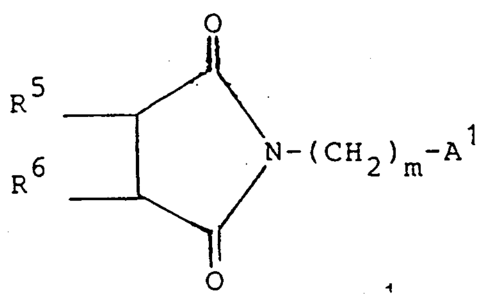

(XVI)

ou un sel de celui-ci (où $A^1$ et m sont tels que définis ci-dessus) et $R^5$ et $R^6$ représentent ensemble un radical de formule XVII, XVIII ou XXI :

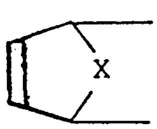

(XVII)

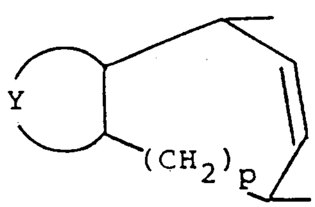

(XVIII)

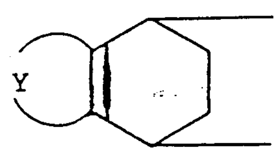

(XXI)

(où X, Y et p sont tels que définis dans la revendication 1 et p n'est pas zéro dans la formule XVIII lorsque Y est $-(CH_2)_o-$ où o est zéro) est réduit pour convertir un composé éthyléniquement insaturé en un composé saturé correspondant; ou
(g) un composé de formule I est converti en un sel de celui-ci par addition d'un acide ou un sel d'un composé de formule I converti en un composé de formule I par soustraction d'un acide.

**2.** Procédé suivant la revendication 1, dans lequel $R^3$ est 2-pyrazinyle ou 3-pyridazinyle non substitué ou substitué, les substituants étant tels que définis dans la revendication 1.

**3.** Procédé suivant la' revendication 1, dans lequel R représente $-CHR^1-CHR^2$ où $R^1$ et $R^2$ représentent ensemble la formule IV telle que définie et illustrée dans la revendication 1 et o est autre que zéro.

**4.** Procédé de préparation d'une composition pharmaceutique, qui comprend la mise en association ou combinaison d'un composé de formule I tel qu'illustré et défini dans la revendication 1 ou d'un sel pharmaceutiquement acceptable de celui-ci avec un excipient pharmaceutiquement acceptable.

**5.** Procédé suivant la revendication 4, dans lequel $R^3$ est tel que défini dans la revendication 2.

**6.** Procédé suivant la revendication 4, dans lequel R est tel que défini dans la revendication 3.

**7.** Procédé suivant l'une quelconque des revendications 4 à 6, dans lequel la composition pharmaceutique est préparée sous forme dosée unitaire comme agent antipsychotique.

**8.** Procédé suivant la revendication 1 ou 4, dans lequel le composé de formule I est la 2-[4-[4-(6-chloro-2-pyrazinyl)-1-pipérazinyl]butyl]hexahydro-4,6-éthénocycloprop[f]isoindole-1,3-(2H,3aH)-dione.

**9.** Procédé suivant la revendication 1 ou 4, dans lequel le composé de formule I est la 4,4a,5,5a,6,6a-hexahydro-2-[4-[4-(2-pyrimidinyl)-1-pipérazinyl]butyl]-4,6-éthanocycloprop[f]isoindole-1,3-(2H,3aH)-dione.

**10.** Procédé suivant la revendication 1 ou 4, dans lequel le composé de formule I est la 3a,4,4a,6a,7,7a-hexahydro-2-[4-[4-(2-pyrimidinyl)-1-pipérazinyl]butyl]-4,7-éthéno-1H-cyclobut[f]isoindole-1,3-(2H)-dione.